# EUROPEAN PATENT APPLICATION

(11) **EP 4 393 955 A1**
(43) Date of publication of application: **03.07.2024**
(21) Application number: 22860533.3
(22) Date of filing: 24.08.2022
(51) Int. Cl.: C07K 16/30, C07K 16/46, C07K 19/00, C12N 15/13, C12N 15/64, C12N 15/85, C12N 15/86, C12N 15/863, A61K 39/395, A61P 35/00

(54) **ANTIBODY SPECIFICALLY BINDING TO CD47, RECOMBINANT ONCOLYTIC VIRUS THEREOF AND USE THEREOF**

(30) Priority: 24.08.2021 CN 202110974061
(71) Applicant: Shanghai Sinobay Biotechnology Co., Ltd., Shanghai 201506 (CN)
(72) Inventor: XU, Jianqing, Shanghai 201506 (CN); ZHANG, Xiaoyan, Shanghai 201506 (CN); DING, Xiangqing, Shanghai 201506 (CN); LIAO, Qibin, Shanghai 201506 (CN); ZHANG, Dan, Shanghai 201506 (CN)
(74) Representative: Uexküll & Stolberg
(86) International application number: PCT/CN2022/114475
(87) International publication number: WO 2023/025187

(57) **Abstract**

Provided is an antibody capable of specifically binding to CD47 or an antigen binding fragment thereof. Also provided is a recombinant oncolytic virus, which is operably inserted with or comprises a gene coding sequence of an anti-CD47 antibody or a CD47 ligand, wherein the anti-CD47 antibody comprises an Fc mutant having A330L/I332E mutations (ALIE antibody), i.e., the anti-CD47 antibody is αCD47-Fc(ALIE).Also provided are a preparation method for the recombinant oncolytic virus, a use of the recombinant oncolytic virus in preparation of anti-tumor drugs, and a vaccinia virus Tiantan strain capable of efficiently expressing an αCD47-Fc(ALIE) gene.

## Description

### Technical Field

The present invention belongs to the technical field of biomedicine and relates to an antibody capable of specifically binding to CD47 or an antigen binding fragment thereof. The present invention also relates to a recombinant oncolytic virus. The present invention further provides a preparation method for the antibody or the antigen binding fragment thereof, a preparation method for the oncolytic virus, and a use of the oncolytic virus in an anti-tumor aspect.

### Background Art

CD47 was first discovered in the 1980s as a tumor antigen of ovarian cancer. Since then, CD47 has been found to be expressed on a variety of human tumors, including acute myeloid leukemia (AML), chronic myeloid leukemia, acute lymphoblastic leukemia (ALL), non Hodgkin lymphoma (NHL), multiple myeloma (MM), bladder cancer and other solid tumors. CD47, also known as an integrin-associated protein (IAP), is a transmembrane protein encoded by a CD47 gene in human bodies. It belongs to the immunoglobulin superfamily and may interact with integrins, thrombospondin (TSP-1), and signal regulatory protein α (SIRPα). CD47, as a signaling molecule that may prevent macrophage phagocytosis, is widely expressed in human cells and is overexpressed in a variety of different cancer cells, achieving a potential to be a therapeutic target for some cancers.

The SIRPα (signal regulatory protein α) is also a transmembrane protein that is mainly expressed on surfaces of macrophages, dendritic cells, and nerve cells. The CD47 highly expressed on surfaces of tumor cells release a signal of "Don't Eat Me" by binding to SIRPα on the surfaces of macrophages, such that macrophages in an infiltration area of tumor tissues not only live in harmony with tumor cells, but also promote the amplification and growth of tumor cells by promoting the proliferation of blood vessels in tumors and inhibiting effector T cells to take effect. Therefore, by blocking an inhibitory signaling pathway transmitted by the binding of CD47 on the surfaces of tumor cells to SIRPα, a phagocytosis function of macrophages to tumor cells can be restored or strengthened, and macrophage-mediated cellular immune responses can be promoted, thereby providing a new theory and method for tumor immunotherapy.

In recent years, a large number of studies have been conducted on various therapeutic methods for a CD47/SIRPα signaling pathway, such as an anti-CD47 antibody, an anti-SIRPα antibody, a recombinant SIRPα protein, and a bispecific antibody. A CD47-blocking antibody is considered to be the most promising therapeutic regimen for tumors. The effectiveness of a human CD47-blocking monoclonal antibody has been demonstrated in a variety of preclinical models, such as lymphoma, bladder cancer, colon cancer, glioblastoma, breast cancer, acute lymphoblastic leukemia, and acute myeloid leukemia. Relevant studies have also confirmed that the use of the anti-CD47 monoclonal antibody in vivo to specifically bind to a highly expressed CD47 protein on the surfaces of tumor cells can effectively relieve an inhibitory effect of the tumor cells on immune cells, restore functions of immune cells in the body, and achieve an effect of inhibiting tumor growth.

Since the discovery that CD47 may be used as a target for next-generation tumor immunotherapy, drug development for this target has never stopped. However, in the process of developing CD47-targeted drugs, the CD47 antibody is prone to cause obvious side effects such as anemia, hyperbilirubinemia and thrombocytopenia, which have caused many studies to hit a wall and stop relevant research trials. This is due to the fact that CD47 is also widely expressed on surfaces of red blood cells, and the binding of a CD47 antibody drug or SIRPα-Fc fusion protein to the red blood cells causes the agglutination of the red blood cells and then triggers the rupture of the red blood cells. At the same time, its Fc segment-mediated cytotoxicity further triggers the lysis of the red blood cells, which ultimately leads to the occurrence of anemia. In addition to obvious anemia and other side effects, the CD47 antibody still has the problems of insufficient antibody affinity, immunogenicity, anti-tumor effect, etc., so the development of candidate antibodies for this target with better effects is urgently needed.

In recent years, the researches on oncolytic viruses for treating tumors have received great attention in the field. Because the oncolytic virus is well targeted to a tumor by itself or by genetic modification, it can locally infect and lyse tumor cells in the tumor. In addition, the oncolytic virus may act on a plurality of cellular pathways, thereby reducing the tumor resistance and also inducing different forms of cell death. At the same time, the oncolytic virus breaks through the immune tolerance of a tumor microenvironment and induces long-acting tumor-specific immune responses. With the help of the oncolytic virus, an antibody protein of CD47 is transported to tumor tissues, and an anti-tumor biological effect of the CD47 antibody can be exerted to effectively avoid the risk of anemia caused by the binding of the CD47 antibody to peripheral red blood cells, and increase the safety of tumor immunotherapy. A plurality of anti-tumor effects may also be exerted in combination with the oncolytic virus, thereby significantly improving an effect of tumor immunotherapy.

The research on a oncolytic virus for treating a tumor has received great attention in the field, and has the following advantages: the oncolytic virus can cooperatively kill tumor cells through a variety of mechanisms to reduce the tumor resistance; the oncolytic virus can play an immunoregulatory role, break through the immune tolerance of the tumor microenvironment, and then induce long-acting tumor-specific immune responses; and the oncolytic virus can transport a therapeutic protein into tumor tissues and increase an expression level in malignant tumor cells as the virus replicates.

In the 1950s and 1970s, there was a brief boom in clinical research and treatment of oncolytic viruses, and initial oncolytic viruses used were of a wild type, which, although showed certain anti-tumor effects in clinical trials, at the same time, also induced excessive immune responses and complications, so that the research and development of the oncolytic viruses in the field were also interrupted. The development of a genetic engineering technology in the 1990s has accelerated the genetic modification and optimization of oncolytic viruses, which has greatly improved the specificity, effectiveness and safety of the oncolytic viruses in the treatment of tumors, and this therapy has once again received extensive attention from researchers. At present, the main types of oncolytic viruses are: an adenovirus, a herpes simplex virus-1, a Newcastle disease virus, a measles virus, a reovirus, a vaccinia virus, etc., among which the adenovirus, the herpes simplex virus and the vaccinia virus are the most widely used in clinical applications.

In 2006, an oncolytic adenovirus product (oncorine) has been used in the clinical treatment of nasopharyngeal carcinoma in China. This oncolytic virus may replicate and proliferate in cancer cells with p53 gene mutations and kill host cells to produce an oncolytic treatment effect by deleting an E1B-SSkD gene of human adenovirus type 5, and meanwhile, allow tumor antigen information to be transmitted by dendritic cells to activate the T cell immunity by deleting an E3 region. However, clinical data showed that compared with radiotherapy, the oncolytic virus oncorine combination chemotherapy had a weaker therapeutic effect on patients with nasopharyngeal carcinoma.

In 2015, Amgen's oncolytic herpes simplex virus (talimogene laherparepvec, T-VEC) was approved by the US FDA for the treatment of melanoma, and in December of the same year, was approved by the European Union for the local treatment of unresectable skin, subcutaneous and lymph node lesions in patients with melanoma that recurred after the first surgery. Clinical research results of T-VEC have greatly promoted the research and development progress of oncolytic viruses in the field of tumor treatment. However, the therapeutic type is limited by intratumoral administration to be used only in tumor types that are close to the body surface and are convenient for surgeries, resulting in problems of difficult administration and incomplete treatment for the treatment of many non-superficial solid tumors and metastases. If it can be proved that intravenous administration still has a good tumor treatment effect, its clinical application value can be greatly increased. Due to the limited oncolytic effect of a herpes virus itself, large and/or metastatic tumors cannot be cleared completely, so it is also necessary to combine other treatment means to enhance the anti-tumor effect.

A vaccinia virus (W) has relatively clear biological traits and pathogenic mechanisms, which play a key role in the elimination of smallpox, and its safety in human bodies has also been fully proven. According to pathogenicity, a host range and other characteristics, vaccinia viruses may be divided into a WR (Western reserve) strain, a Wyeth strain, a Copenhagen strain, a Lister strain, a Tian Tan strain, etc. Because of their wide host ranges, high conservatism, good safety and large capacity of exogenous genes, the vaccinia viruses have been used as vectors for many recombinant vaccines such as an influenza virus and a human immunodeficiency virus. Most of the research and development of oncolytic viruses are currently in a preclinical research stage, and only a few have entered a clinical research stage.

Among researches on the use of vaccinia viruses as oncolytic viruses for the treatment of tumors, the fastest progress at present is Pexa-Vec (JX-594) developed by Jennerex in the United States. JX-594 is based on the Wyeth strain, and hGM-CSF and LacZ genes are inserted into a TK region. Due to the deletion of a thymic kinase gene, JX-594 may be expressed and replicated in cancer cells expressing thymus kinase at a high level, but cannot affect normal cells. At the same time, JX-594 may express GM-CSF in tumor cells due to the insertion of a GM-CSF gene, and activate an anti-tumor immune response of the body. The results of clinical trials of JX-594 in a plurality of tumor types demonstrated that it was well tolerated by intratumoral administration or intravenous drip, and the effect of Pexa-Vec combined with sorafenib was better than that of an administration group alone. Interim analysis results showed that it was not likely to prolong the patient lifetime. Pexa-Vec, which was originally scheduled to be launched in 2020, was terminated early in the Phase III clinical trial.

An oncolytic virus (GL-ONC1, also known as GLU-1h68) developed by Genelux in the United States is based on a vaccinia virus (Lister strain). The targeting of a tumor can be strengthened by deleting its genes F14.5L, TK (encoding thymidine kinase) and HA (encoding hemagglutinin). Poxviruses can be screened, produced and prepared by inserting a luciferase-GFP fusion protein, a β-galactosyltransferase and a β-glucuronidase respectively. A completed Phase I clinical trial of GL-ONC1 for intravenous administration had shown good safety and efficacy, without dose-limiting toxicity, no maximum tolerated dose was reached, and all patients had a neutralizing response to GL-ONC1. The GL-ONC1 is currently undergoing a scale-up trial for intravenous administration.

Clinical trial evidences showed that a vaccinia virus had shown some initial advantages in the treatment of tumors. Most of the existing designs using vaccinia viruses as oncolytic viruses are safe, and the clinical effectiveness needs to be further observed, but further optimization is also needed in immunoregulation and precise tumor targeting. How to use oncolytic viruses to reconstruct a tumor microenvironment, enhance immune responses, improve the lethality to tumor cells, and realize the use of oncolytic viruses alone is still an urgent problem to be solved in the research and development of oncolytic viruses. How to maximize the advantages of CD47 targets in killing tumor cells while reducing the damage to red blood cells has always been the key to the development of CD47-targeting drugs. Existing strategies include screening SIRPα molecules that bind only to CD47 on the surfaces of tumor cells, such as an Fc fusion protein drug IMM01. An IgG1 antibody that was screened by IMMUNEONCO and did not bind to human erythrocytes at all had been approved for clinical trials for the treatment of hematological malignancies in China in May 2019 and were currently underway.

Another strategy was to replace an IgG1 Fc segment with an IgG4 Fc segment, such as an antibody drug Hu5F9-G4, and a strategy of low-dose induction + effective dose maintenance and combination with rituximab was used to alleviate side effects of anemia and had shown good efficacy in clinical studies of patients with aggressive and indolent lymphoma. However, there was still a risk of anemia caused by the binding of CD47 on surfaces of red blood cells in peripheral blood to a CD47 antibody, while Hu5F9-G4 alone has little effect.

If the CD47 antibody is delivered directly to tumor cells through a virus vector, it is possible to avoid the occurrence of anemia. Some studies have been conducted to construct a single-chain antibody (scFv) by conjugating a light chain and a heavy chain of the CD47 antibody expressed by a recombinant poxvirus, so as to recognize a CD47 antigen of tumor cells, thereby exerting an oncolytic effect more accurately. At the same time, a blocking effect of CD47 was used to enhance the phagocytosis of macrophages on tumors. Clinical research results have shown that a recombinant oncolytic vaccinia virus (OVVscFvCD47) has a better anti-lymphoma effect. Since the prerequisite for a CD47-targeting antibody drug to exert a highly effective anti-tumor effect is to fully activate macrophages, two basic conditions must be met: 1) CD47-SIRPα interaction is blocked by the CD47 antibody or SIRPα-Fc to release an inhibiting signal; and 2) a Fc terminal of an antibody IgG1 binds to a Fcy receptor on the surface of a macrophage membrane, resulting in the allosterism of a macrophage membrane skeleton to increase the phagocytic activity. Otherwise, macrophages can only be partially activated. At the same time, in the lack of an Fc segment and an ADCC effect, a single drug will have no good therapeutic effect and must be used in combination with other antibody drugs. In particular, the short half-life of scFv in vivo greatly weakens its anti-tumor effect.

Based on the above, there is no CD47 antibody that is particularly suitable for a recombinant oncolytic virus in the prior art, and there is a demand for a CD47 antibody with better tumor cell killing ability and an oncolytic virus that can realize the independent use of the oncolytic virus.

### Summary of the Invention

Therefore, an object of the present invention is to provide an antibody capable of specifically binding to CD47 in view of the defects of the prior art. The anti-CD47 antibody provided by the present invention has strong specificity, high affinity, low immunogenicity, and good stability, can be used to prevent or treat cancers, and has a broad application prospect. The present invention further provides a new recombinant oncolytic virus. The oncolytic virus provided by the present invention comprises a gene coding sequence of the anti-CD47 antibody or a CD47 ligand, which is capable of replicating in large numbers within tumor cells and ultimately destroying tumor cells. Specifically, the recombinant oncolytic virus provided by the present invention comprises a gene coding sequence of the anti-CD47 antibody. The gene sequence of the anti-CD47 antibody of the present invention comprises a mutated Fc segment, which has high killing activity and can significantly enhance an anti-tumor effect using a strong ADCC effect mediated by the antibody.

The objects of the present invention are achieved by the following technical solutions.

In an aspect, the present invention provides an antibody capable of specifically binding to CD47 or an antigen binding fragment thereof, the antibody or the antigen binding fragment comprising:
(a) a heavy chain variable region comprising the following three complementary determining regions:
   (i) VH CDR1, which consists of the following sequence: SEQ ID NO: 17, or a sequence having one or more amino acid substitutions, deletions, or additions compared thereto;
   (ii) VH CDR2, which consists of the following sequence: SEQ ID NO: 18, or a sequence having one or more amino acid substitutions, deletions, or additions compared thereto; and
   (iii) VH CDR3, which consists of the following sequence: SEQ ID NO: 19, or a sequence having one or more amino acid substitutions, deletions, or additions compared thereto;
   and/or
(b) a light chain variable region comprising the following three complementary determining regions:
   (iv) VL CDR1, which consists of any one of the following sequences: SEQ ID NO: 11, SEQ ID NO: 14, or SEQ ID NO: 22, or a sequence having one or more amino acid substitutions, deletions, or additions compared thereto;
   (v) VL CDR2, which consists of any one of the following sequences: SEQ ID NO: 12, SEQ ID NO: 15, or SEQ ID NO: 23, or a sequence having one or more amino acid substitutions, deletions, or additions compared thereto; and
   (vi) VL CDR3, which consists of any one of the following sequences: SEQ ID NO: 13, SEQ ID NO: 16, or SEQ ID NO: 24, or a sequence having one or more amino acid substitutions, deletions, or additions compared thereto;

preferably, the substitution in any one of (i) to (vi) is a conservative substitution; and
preferably, the VH in the antibody or the antigen binding fragment thereof comprises: VH CDR1 as shown in SEQ ID NO: 17, VH CDR2 as shown in SEQ ID NO: 18, and VH CDR3 as shown in SEQ ID NO: 19; and the VL in the antibody or the antigen binding fragment thereof comprises: VL CDR1 as shown in SEQ ID NO: 11, VL CDR2 as shown in SEQ ID NO: 12, and VL CDR3 as shown in SEQ ID NO: 13; or the VL in the antibody or the antigen binding fragment thereof comprises: VL CDR1 as shown in SEQ ID NO: 14, VL CDR2 as shown in SEQ ID NO: 15, and VL CDR3 as shown in SEQ ID NO: 16; or the VL in the antibody or the antigen binding fragment thereof comprises: VL CDR1 as shown in SEQ ID NO: 22, VL CDR2 as shown in SEQ ID NO: 23, and VL CDR3 as shown in SEQ ID NO: 24.

The present invention provides an antibody capable of specifically binding to CD47 or an antigen binding fragment thereof, the antibody or the antigen binding fragment thereof comprising a heavy chain variable region and a light chain variable region, wherein:
the heavy chain variable region comprises three CDRs contained in the heavy chain variable region shown in SEQ ID NO: 9; and the light chain variable region comprises three CDRs contained in the light chain variable region shown in SEQ ID NO: 5, 7, or 25; and
preferably, the three CDRs contained in the heavy chain variable region, and/or the three CDRs contained in the light chain variable region, are defined by a Kabat, Chothia, or IMGT numbering system.

The antibody or the antigen binding fragment thereof according to the present invention comprises:
(a) a heavy chain variable region comprising an amino acid sequence selected from the group consisting of:
   (i) a sequence shown in SEQ ID NO: 9;
   (ii) a sequence having one or more amino acid substitutions, deletions, or additions compared to the sequence shown in SEQ ID NO: 9; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence shown in SEQ ID NO: 9;
   and/or,
(b) a light chain variable region comprising an amino acid sequence selected from the group consisting of:
   (iv) a sequence shown in any one of SEQ ID NO: 5, SEQ ID NO: 7, or SEQ ID NO: 25;
   (v) a sequence having one or more amino acid substitutions, deletions, or additions compared to the sequence shown in any one of SEQ ID NO: 5, SEQ ID NO: 7, or SEQ ID NO: 25; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence shown in any one of SEQ ID NO: 5, SEQ ID NO: 7, or SEQ ID NO: 25;

preferably, the substitution in (ii) or (v) is a conservative substitution;
preferably, the antibody or the antigen binding fragment thereof comprises: a VH having a sequence as shown in SEQ ID NO: 9 and a VL having a sequence as shown in any one of SEQ ID NO: 5, 7, or 25.

The antibody or the antigen binding fragment thereof according to the present invention further comprises:
(a) a heavy chain constant region of a human immunoglobulin or a variant thereof, the variant having one or more amino acid substitutions, deletions, or additions compared to a sequence from which the variant is derived; and
(b) a light chain constant region of a human immunoglobulin or a variant thereof, the variant having conservative substitutions of up to 20 amino acids compared to a sequence from which the variant is derived;

preferably, the heavy chain constant region is an IgG heavy chain constant region, more preferably an IgG1, IgG2, IgG3, or IgG4 heavy chain constant region, further preferably, a human IgG1 or human IgG4 heavy chain constant region; and
preferably, the light chain constant region is a κ light chain constant region.

In the antibody or the antigen binding fragment thereof according to the present invention, the antigen binding fragment is selected from the group consisting of Fab, Fab', (Fab')₂, Fv, disulfide-bonded Fv, scFv, a double antibody, and a single-domain antibody; and/or, the antibody is a murine antibody, a chimeric antibody, a humanized antibody, a bispecific antibody, or a multi-specific antibody; and more preferably, the antibody is a fully human antibody.

In another aspect, the present invention provides a chimeric antigen receptor T cell, comprising the antibody or the antigen binding fragment thereof of the present invention; and
preferably, the heavy chain variable region and the light chain variable region in the antibody or the antigen binding fragment thereof are combined in series or in parallel.

The present invention further provides an isolated nucleic acid molecule, which encodes the antibody capable of specifically binding to CD47 or the antigen binding fragment thereof according to the present invention, or a heavy chain variable region and/or a light chain variable region thereof; and
preferably, the nucleic acid molecule comprises a nucleic acid sequence shown in any one of SEQ ID NO: 6, 8, 10, or 26.

The present invention further provides a vector, comprising the isolated nucleic acid molecule according to the present invention;
preferably, the vector is a cloning vector or an expression vector;
more preferably, the vector is a virus; and
further preferably, the vector is a cloning vector AbVec-hIgKappa or a cloning vector AbVec-hIgG1.

The present invention further provides a host cell, comprising the isolated nucleic acid molecule and the vector according to the present invention; and
preferably, the host cell is prokaryotic or eukaryotic; more preferably, the host cell is selected from an E. coli cell, a yeast cell, a mammalian cell or other cells suitable for the preparation of an antibody or an antigen binding fragment, or a multi-specific antibody; further preferably, the host cell is a mammalian cell; further preferably, the host cell is a cell derived from human beings, mouse, sheep, horse, dog, or cat; and most preferably, the host cell is a 293 cell or a CHO cell.

In yet another aspect, the present invention provides a method for preparing the antibody or the antigen binding fragment thereof according to the present invention, comprising: culturing the host cell under a condition that allows the expression of the antibody or the antigen binding fragment thereof according to the present invention; and recycling the antibody or the antigen binding fragment thereof from the cultured host cell culture.

In a further aspect, the present invention provides a bispecific or multi-specific molecule, comprising the antibody or the antigen binding fragment thereof according to the present invention;
preferably, the bispecific or multi-specific molecule specifically binds to CD47 and additionally specifically binds to one or more other targets;
preferably, the bispecific or multi-specific molecule further comprises at least one molecule (e.g., a secondary antibody) with a second binding specificity for a second target; and
preferably, the bispecific or multi-specific molecule further comprises other antibodies that specifically bind to a CD47 epitope or an antigen binding fragment thereof.

In a further aspect, the present invention provides an immunoconjugate, comprising the antibody or the antigen binding fragment thereof according to the present invention and a therapeutic agent ligated to the antibody or the antigen binding fragment thereof;
preferably, the therapeutic agent is selected from a cytotoxic agent;
preferably, the therapeutic agent is selected from an alkylating agent, a mitotic inhibitor, an anti-tumor antibiotic, an antimetabolite, a topoisomerase inhibitor, a tyrosine kinase inhibitor, a radionuclide agent, and any combination thereof; and
preferably, the immunoconjugate is an antibody-drug conjugate (ADC).

In a further aspect, the present invention provides a pharmaceutical composition, comprising the antibody or the antigen binding fragment thereof, the bispecific or multi-specific molecule or the immunoconjugate according to the present invention, and a pharmaceutically acceptable carrier and/or excipient;
preferably, the pharmaceutical composition further comprises an additional pharmaceutically active agent;
preferably, the additional pharmaceutically active agent is a agent with anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizing agent, an anti-angiogenic agent, a cytokine, a molecularly targeted agent, an immune checkpoint inhibitor, or an oncolytic virus; and
preferably, the antibody or the antigen binding fragment thereof, and the bispecific or multi-specific molecule, or the immunoconjugate are provided as separate components or as components of the same composition with the additional pharmaceutically active agent.

In a further aspect, the present invention provides a kit, comprising the antibody or the antigen binding fragment thereof according to the present invention;
preferably, the antibody or the antigen binding fragment thereof carries a detectable marker, such as an enzyme (e.g., horseradish peroxidase), a radionuclide, a fluorescent dye, a luminescent substance (such as a chemiluminescent substance), or a biotin;
preferably, the kit further comprises a secondary antibody which specifically recognizes the antibody or the antigen binding fragment thereof according to the present invention; and
preferably, the secondary antibody further comprises a detectable marker, such as an enzyme (e.g., horseradish peroxidase), a radionuclide, a fluorescent dye, a luminescent substance (such as a chemiluminescent substance), or a biotin.

In a further aspect, the present invention provides a chimeric antigen receptor, comprising an antigen binding domain of the antibody or the antigen binding fragment thereof according to the present invention;
preferably, the antigen binding domain comprises a heavy chain variable region and a light chain variable region of the antibody or the antigen binding fragment thereof according to the present invention;
preferably, the antigen binding domain is scFv;
preferably, the antigen binding receptor comprises the antigen binding fragment of the antibody according to the present invention; and
preferably, the antigen binding receptor is expressed by an immune effector cell (e.g., a T cell).

In a further aspect, the present invention provides an isolated nucleic acid molecule, which encodes the chimeric antigen receptor.

In a further aspect, the present invention provides a vector, which comprises an isolated nucleic acid molecule encoding the chimeric antigen receptor, and preferably, is used for the preparation of a chimeric antigen receptor T cell.

In a further aspect, the present invention provides a host cell, which comprises an isolated nucleic acid molecule or a vector that encodes the chimeric antigen receptor;
preferably, the host cell is an immune effector cell (e.g., a T cell or a NK cell); and
preferably, the host cell is a chimeric antigen receptor T cell (CAR-T).

The present invention further provides a method for inhibiting the growth of a tumor cell and/or killing the tumor cell, which comprises: allowing the tumor cell to contact with an effective amount of the antibody or the antigen binding fragment thereof, the bispecific or multi-specific molecule, the immunoconjugate, the pharmaceutical composition, the chimeric antigen receptor, or the host cell of the present invention.

The present invention further provides a method for the prevention and/or treatment of a tumor in a subject (e.g., human), comprising administrating to the subject in need thereof an effective amount of the antibody or the antigen binding fragment thereof, the bispecific or multi-specific molecule, the immunoconjugate, the pharmaceutical composition, the chimeric antigen receptor, or the host cell according to the present invention;
preferably, the tumor is selected from B-cell lymphoma; T-cell lymphoma; melanoma; prostatic cancer; renal cell carcinoma; sarcoma; glioma, such as high-grade glioma; blastoma, such as neuroblastoma; osteosarcoma; plasmacytoma; histiocytoma; pancreatic cancer; breast cancer; lung cancers such as small cell lung cancer and non-small cell lung cancer; gastric cancer; liver cancer; colon cancer, rectal cancer; esophageal cancer; colorectal cancer; hematopoietic system cancer; testicular cancer; cervical cancer; ovarian cancer; bladder cancer; squamous cell carcinoma; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or blood neoplastic disorders;
preferably, the subject is a mammal, such as human;
preferably, the method further comprises: administrating an additional agent with anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizing agent, an anti-angiogenic agent, a cytokine, a molecularly targeted agent, an immune checkpoint inhibitor, or an oncolytic virus; and
preferably, the method further comprises: administrating an additional antineoplastic therapy, such as a surgery, a chemotherapy, a radiotherapy, a targeted therapy, an immunotherapy, a hormonal therapy, a gene therapy, or a palliative care.

The present invention further provides use of the antibody or the antigen binding fragment thereof, the bispecific or multi-specific molecule, the immunoconjugate, the pharmaceutical composition, the chimeric antigen receptor, or the host cell according to the present invention in the preparation of a medicament, the medicament being used for the prevention and/or treatment of a tumor in a subject (e.g., human);
preferably, the medicament further comprises an additional pharmaceutically active agent;
preferably, the additional pharmaceutically active agent is a agent with anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizing agent, an anti-angiogenic agent, a cytokine, a molecularly targeted agent, an immune checkpoint inhibitor, or an oncolytic virus; and
preferably, the tumor is selected from B-cell lymphoma; T-cell lymphoma; melanoma; prostatic cancer; renal cell carcinoma; sarcoma; glioma, such as high-grade glioma; blastoma, such as neuroblastoma; osteosarcoma; plasmacytoma; histiocytoma; pancreatic cancer; breast cancer; lung cancers such as small cell lung cancer and non-small cell lung cancer; gastric cancer; liver cancer; colon cancer, rectal cancer; esophageal cancer; colorectal cancer; hematopoietic system cancer; testicular cancer; cervical cancer; ovarian cancer; bladder cancer; squamous cell carcinoma; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or blood neoplastic disorders; and
preferably, the subject is a mammal, such as human.

In another aspect, the present invention further provides a recombinant oncolytic virus, which is operably inserted with or comprises a gene coding sequence of an anti-CD47 antibody or a CD47 ligand.

Preferably, the gene coding sequence is located in a thymidine kinase (TK) region of the recombinant oncolytic virus.

Preferably, a gene coding sequence of the anti-CD47 antibody or the CD47 ligand may be expressed alone, or may also be expressed in fusion with other genes or fragments; and more preferably, other genes or fragments for fusion expression include, but are not limited to one or more of a Fc fragment, a chemokine CXCL9, CXCL10, CXCL11, CXCL12, CCL20 or CX3CL1, or a cholera toxin CTA or CTB.

Preferably, the recombinant oncolytic virus further comprises a gene coding sequence of other immunoregulatory factors; more preferably, the other immunoregulatory factors include, but are not limited to IL-1, IL-2, IL-3, IL-7, IL-11, IL-12, IL-15, IL-17, IL-18, IL-21, IL-33, IL-35, IL-37, GM-CSF, IFN-α, IFN-β, IFN-γ, an anti-PD-1/PD-L1 antibody, an anti-CTLA-4 antibody, an anti-Lag-3 antibody, an anti-TIGIT antibody, or an anti-Tim-3 antibody; or
the recombinant oncolytic virus further comprises a gene coding sequence of a protein related to apoptosis and pyroptosis; the protein related to apoptosis and pyroptosis includes, but is not limited to an apoptosis-related factor 1 (Apaf-1), an interleukin-1β converting enzyme (ICE), a Bcl-2 protein, Fas/APO-1, p53, myc, an ataxia telangiectasia mutant (ATM) gene, gasdermin D, gasdermin E, etc.; or
the recombinant oncolytic virus further comprises small RNAs of an immunoregulatory gene, an apoptosis gene, and a pyroptosis gene.

In the recombinant oncolytic virus according to the present invention, the anti-CD47 antibody comprises the antibody or the antigen binding fragment thereof as described above; and preferably, the anti-CD47 antibody comprises an Fc mutant (ALIE antibody) having A330L/I332E mutations, i.e., the anti-CD47 antibody is αCD47-Fc(ALIE).

In the recombinant oncolytic virus according to the present invention, the anti-CD47 antibody has a sequence identity of at least 90%, 91%, 92%, 93%, 94%, 95%, 96%, 97%, 98%, or 99% with the sequence shown in SEQ ID NO: 2.

Preferably, an amino acid sequence of the anti-CD47 antibody is shown in SEQ ID NO: 2.

In the recombinant oncolytic virus according to the present invention, the oncolytic virus comprises a gene coding sequence having a sequence identity of at least 90%, 91%, 92%, 93%, 94%, 95%, 96%, 97%, 98%, or 99% with the sequence shown in SEQ ID NO: 1.

Preferably, the oncolytic virus comprises the gene coding sequence as shown in SEQ ID NO: 1;
in the oncolytic virus according to the present invention, the CD47 ligand is a SIRPα extracellular domain, or a functional fragment or variant thereof; and
preferably, the anti-CD47 ligand has a sequence identity of at least 90%, 91%, 92%, 93%, 94%, 95%, 96%, 97%, 98%, or 99% with the sequence shown in SEQ ID NO: 4.

Preferably, the amino acid sequence of the anti-CD47 ligand is shown in SEQ ID NO: 4.

In the recombinant oncolytic virus according to the present invention, the oncolytic virus comprises a gene coding sequence having a sequence identity of at least 90%, 91%, 92%, 93%, 94%, 95%, 96%, 97%, 98%, or 99% with the sequence shown in SEQ ID NO: 3.

Preferably, the oncolytic virus comprises a gene coding sequence as shown in SEQ ID NO: 3.

In the recombinant oncolytic virus according to the present invention, a virus skeleton of the oncolytic virus is derived from a modified or engineered vaccinia virus Tian Tan strain, a vaccinia virus New York strain, a vaccinia virus Copenhagen strain, a vaccinia virus canary strain, a vaccinia virus Ankara strain, an adenovirus, an adeno-associated virus, a herpes simplex virus, a varicella-zoster virus (VZV), a respiratory syncytial virus (RSV), a Semliki forest virus (SFV), an EB virus, a cytomegalovirus, a human Herpesvirus 6, a smallpox virus, a vaccinia virus, a molluscum contagiosum virus, an Orfvirus, a reovirus, a rotavirus, an enterovirus, a Seneca virus, a poliovirus, a coxsackievirus, a rhinovirus, a hepatitis A virus, a foot and mouth disease virus, a togavirus, an alphavirus, a Semliki forest virus, an eastern equine encephalitis virus, a Sindbis virus, a rubella virus, a coronavirus, a flavivirus, a hepatitis C virus, a Japanese Encephalitis virus, a St. Louis encephalitis virus, a Murray Valley fever virus, a yellow fever virus, a West Nile virus, a Zika virus, a dengue virus, an Ebola virus, a Marburg virus, an arenavirus, a Lassa fever virus, a lymphocytes Choriomeningitis virus, a Pichinde virus, a Junin virus, Machupo virus, a Hantaan virus, a Rift Valley fever virus, a Paramyxovirus, a human parainfluenza virus, a Mumps virus, a simian virus 5, a measles virus, a vesicular stomatitis virus, a rabies virus, an orthomyxovirus, an influenza A virus, an influenza B virus, an influenza C virus, a hepatitis D virus, a simian immunodeficiency virus, a human immunodeficiency virus 1, a human immunodeficiency virus 2, a Rous sarcoma virus, a human T-cell leukemia virus 1, a simian foamy virus, a hepatitis B virus, hepatitis E virus, a human papillomavirus, or a polyomavirus.

Preferably, the oncolytic virus skeleton is an intracellular mature virus, an intracellular packaging virus, a cell-associated packaging virus, or an extracellular packaging virus.

In the recombinant oncolytic virus according to the present invention, the oncolytic virus is a recombinant vaccinia virus Tian Tan strain comprising a gene coding sequence as shown in SEQ ID NO: 1, named rTV-αCD47-Fc(ALIE) and having a deposit accession number of CCTCC NO: V202080, a deposit date of January 8, 2021, and a depositary institution of China Center for Type Culture Collection (CCTCC) (address: Wuhan University, Wuhan, China).

In a further aspect, the present invention provides a method for preparing the recombinant oncolytic virus. The method comprises the steps of:
1) synthesizing a sequence including a gene coding sequence of an anti-CD47 antibody or a CD47 ligand;
2) cloning the coding sequence obtained in step 1) into a shuttle plasmid of the oncolytic virus to construct a recombinant plasmid vector; and
3) transfecting the recombinant plasmid vector obtained in step 2) into the oncolytic virus, and obtaining the recombinant oncolytic virus through screening.

Optionally, the obtained recombinant oncolytic virus is cultured.

In a specific embodiment, the present invention provides a method for preparing a recombinant vaccinia virus Tian Tan strain. The method comprises the steps of:
1) synthesizing a human gene αCD47-Fc(ALIE), having a sequence as shown in SEQ ID NO: 1; or
   comprising therein a nucleic acid sequence shown in any one of SEQ ID NO: 6, 8, 10, or 26; or
   synthesizing a human gene SIRPα-Fc(ALIE), having a sequence as shown in SEQ ID NO: 3;
2) subcloning the synthesized αCD47-Fc(ALIE) gene or SIRPα-Fc(ALIE) gene into a TK region of a vaccinia virus shuttle plasmid (pSC65) to construct a recombinant plasmid pSC65-αCD47-Fc(ALIE) or pSC65-SIRPα-Fc(ALIE); and
3) transfecting the pSC65-αCD47-Fc(ALIE) plasmid or the pSC65-SIRPα-Fc(ALIE) plasmid into TK143-cells that have been infected with a wild-type vaccinia virus by means of gene homologous recombination, and homologously recombining the both to produce a recombinant vaccinia virus rTV-αCD47-Fc(ALIE) or rTV-SIRPα-Fc(ALIE); and obtaining through screening a recombinant oncolytic vaccinia virus, of which the TK region includes a coding sequence of pSC65-αCD47-Fc(ALIE) shown in SEQ ID NO: 1, or a recombinant oncolytic vaccinia virus, of which the TK region includes a coding sequence of pSC65-SIRPα-Fc(ALIE) shown in SEQ ID NO: 3.

The αCD47-Fc(ALIE) gene or the SIRPα-Fc(ALIE) gene is controlled by an early/late promoter p7.5 of the vaccinia virus.

Preferably, amplifying the recombinant vaccinia virus using VERO cells specifically comprises the steps of: replacing the culture medium with a low-concentration fetal bovine serum maintenance medium when the VERO cells are cultured to a density close to 100%; adding an oncolytic vaccinia virus (the inoculation amount being about 0.02 MOI) into a 37°C incubator, and culturing and amplifying the same for 48 h; collecting a virus solution and repeatedly freezing and thawing the same twice; and then preforming density gradient centrifugation for purification with a 36% sucrose solution, and storing the same under freezing at -80°C.

In a further aspect, the present invention further provides use of the recombinant oncolytic virus in the preparation of an anti-tumor medicament, wherein the tumor is selected from B-cell lymphoma; T-cell lymphoma; melanoma; prostatic cancer; renal cell carcinoma; sarcoma; glioma, such as high-grade glioma; blastoma, such as neuroblastoma; osteosarcoma; plasmacytoma; histiocytoma; pancreatic cancer; breast cancer; lung cancers such as small cell lung cancer and non-small cell lung cancer; gastric cancer; liver cancer; colon cancer, rectal cancer; esophageal cancer; colorectal cancer; hematopoietic system cancer; testicular cancer; cervical cancer; ovarian cancer; bladder cancer; squamous cell carcinoma; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or blood neoplastic disorders.

In another aspect, the present invention provides a method for the treatment of a tumor. The method comprises: administrating to a subject in need thereof a therapeutically effective amount of a recombinant oncolytic virus. The method according to the present invention further comprises: administrating to a subject in need thereof an additional chemotherapy drug, radiotherapy technology, surgical treatment, immune cell drug (including, but not limited to CAR-T, NK, NKT, iNKT, CAR-NKT, CAR-NKT, CAR-iNKT, etc.), and other oncolytic viruses; and preferably, the method is intravenous injection or intratumoral injection.

An inventive concept of the present invention is as follows: the inventors of the present invention obtained 25 novel strains targeting a CD47 antibody by screening a native bacteriophage antibody library; further purified and expressed the exemplary antibodies Hu004-65, Hu004-66, Hu004-67, Hu004-68, Hu004-69, Hu004-73, Hu004-74, Hu004-91, Hu004-100, and Hu004-101; and identified in vitro biological functions of these antibodies. The inventors surprisingly found that Hu004-65 and Hu001-101 had good biological effects and could recognize a human CD47 protein and had the affinities of 1.46×10⁻⁷ M and 3.20× 10⁻⁷ M. Further, the inventors have found that the antibodies Hu004-65 and Hu004-101 have different recognition epitopes from a control antibody, can effectively recognize and bind to humanized CD47 and murine CD47, have good in vitro broad-spectrum mediated anti-tumor efficacy, and may be used to prepare CART for targeting diseases with high CD47 expression or anti-tumor drugs that form conjugate drugs with cytotoxic drugs, radioisotopes, and drug-metabolizing enzymes, thereby achieving a very good prospect.

Further, the inventors of the present invention combine a gene therapy for tumors with an oncolytic effect, and prepare an oncolytic virus of a vaccinia virus Tian Tan strain that can efficiently express an αCD47-Fc(ALIE) gene or a SIRPα-Fc(ALIE) gene. When the oncolytic virus is delivered to a tumor lesion, it expresses the CD47 antibody while lysing tumor cells to exert an oncolytic effect, and then blocks the binding of CD47 to SIRPα, relieves an inhibitory effect of the tumor cells on macrophages, and improves a phagocytic function of the macrophages on the tumor cells. At the same time, an ADCC effect mediated by the CD47 antibody is used to enhance an anti-tumor effect. Compared with a simple antibody therapy or virus therapy, the oncolytic virus significantly enhances an inhibition ability on malignant tumors, thereby significantly improving the effect of tumor immunotherapy. Meanwhile, the risk of anemia caused by the binding of the CD47 antibody to peripheral red blood cells is effectively avoided to increase the safety of tumor immunotherapy.

The present invention has the following beneficial effects.
1. The antibodies Hu004-65 and Hu004-101 provided by the present invention have different recognition epitopes from a control antibody, can effectively recognize and bind to humanized CD47 and murine CD47, have good in vitro broad-spectrum mediated anti-tumor efficacy, and may be used to prepare CART for targeting diseases with high CD47 expression or anti-tumor drugs that form conjugate drugs with cytotoxic drugs, radioisotopes, and drug-metabolizing enzymes, thereby achieving a very good prospect.
2. The recombinant oncolytic virus provided by the present invention combines a gene therapy for tumors with an oncolytic effect. Specifically, the present invention provides an oncolytic virus of a vaccinia virus Tian Tan strain that can efficiently express an αCD47-Fc(ALIE) gene or a SIRPα-Fc(ALIE) gene. When the oncolytic virus is delivered to a tumor lesion, it expresses the CD47 antibody while lysing tumor cells to exert an oncolytic effect, and then blocks the binding of CD47 to SIRPα, relieves an inhibitory effect of the tumor cells on macrophages, and improves a phagocytic function of the macrophages on the tumor cells. At the same time, an ADCC effect mediated by the CD47 antibody is used to enhance an anti-tumor effect. Compared with a simple antibody therapy or virus therapy, the oncolytic virus significantly enhances an inhibition ability on malignant tumors, thereby significantly improving the effect of tumor immunotherapy. Meanwhile, the risk of anemia caused by the binding of the CD47 antibody to peripheral red blood cells is effectively avoided to increase the safety of tumor immunotherapy.
3. The present invention has completed the in vitro experimental research on the treatment of liver cancer, malignant lung cancer, etc. by the oncolytic virus of the vaccinia virus Tian Tan strain, has achieved good targeting and anti-tumor effects on tumors, and has a relatively complete virus amplification and quality control system, which lays the foundation for further industrialization, and has a good application prospect.
4. The recombinant vaccinia virus Tian Tan strain provided by the present invention is innovatively loaded with a CD47 antibody gene with a strong ADCC effect. CD47 molecules are used to precisely target tumor cells, with the superposition of an anti-tumor biological effect of the CD47 antibody. While the oncolytic virus exerts an oncolytic effect and lyses tumor cells, it also kills local tumor cells by expressing a large amount of αCD47-Fc(ALIE), thereby exerting multiple anti-tumor effects. Compared with a single gene therapy or virus therapy, the ability to kill malignant tumors is enhanced. Therefore, the effect of tumor immunotherapy is promoted significantly. Meanwhile, the risk of anemia caused by the binding of the CD47 antibody to peripheral red blood cells is effectively avoided to increase the safety of tumor immunotherapy.

### Brief Description of the Drawings

The embodiments of the present invention will be described in detail below with reference to the accompanying drawings. In drawings:

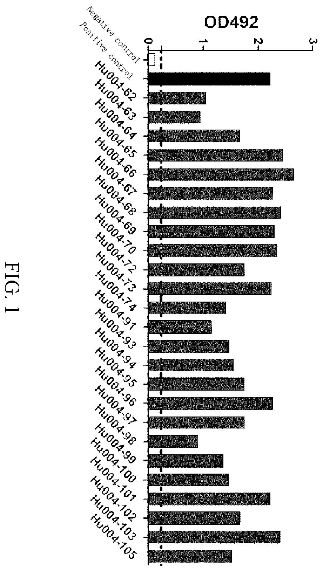

FIG. 1 shows a histogram of the binding of a supernatant of a clone screened by ELISA to an antigen hCD47-ECD according to Example 1 of the present invention, and the result shows that there are 25 strains of candidate antibody clones that can specifically bind to hCD47-ECD, wherein monoclonal antibodies of Hu004-65 and Hu004-101 according to the present invention have higher specificity.

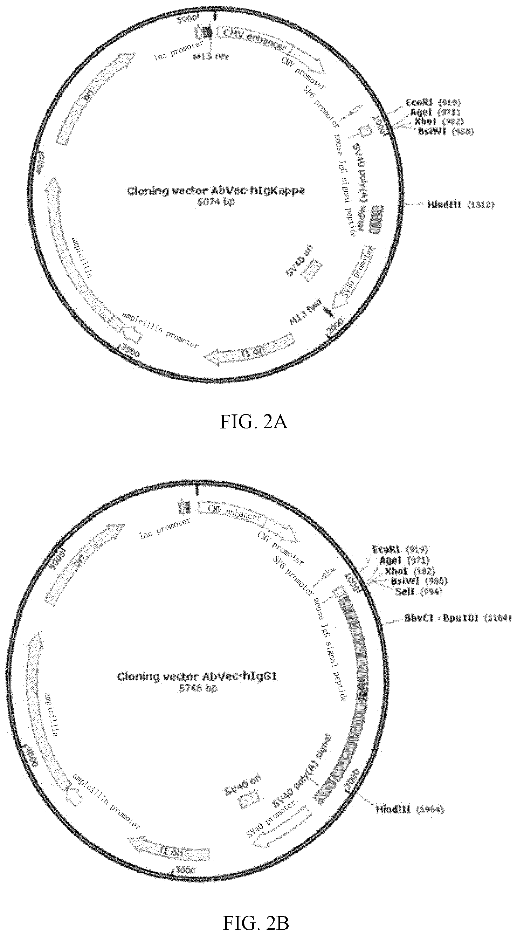

FIG. 2 represents an expression vector map for an antibody of the present invention, wherein FIG. 2A is Cloning vector AbVec-hIgKappa, and FIG. 2B is Cloning vector AbVec-hIgG1.

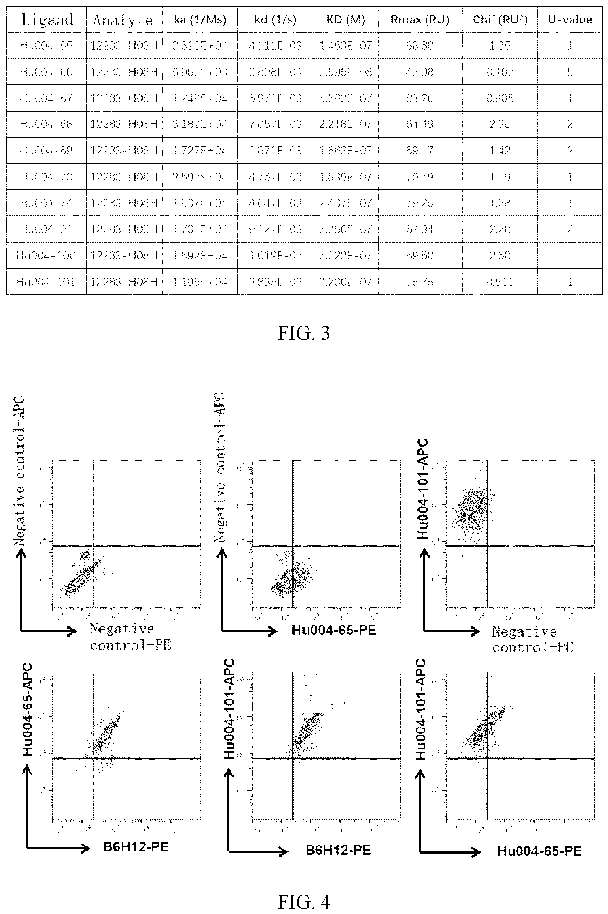

FIG. 3 shows the affinity of an antibody of the present invention which is determined according to a surface plasmon resonance method (SPR), and the result shows that 10 strains of antibodies have stronger affinity.
FIG. 4 shows that monoclonal antibodies Hu004-65 and Hu004-101 of the present invention have different recognition epitopes from the control antibody B6H12 (US 9017675B2).

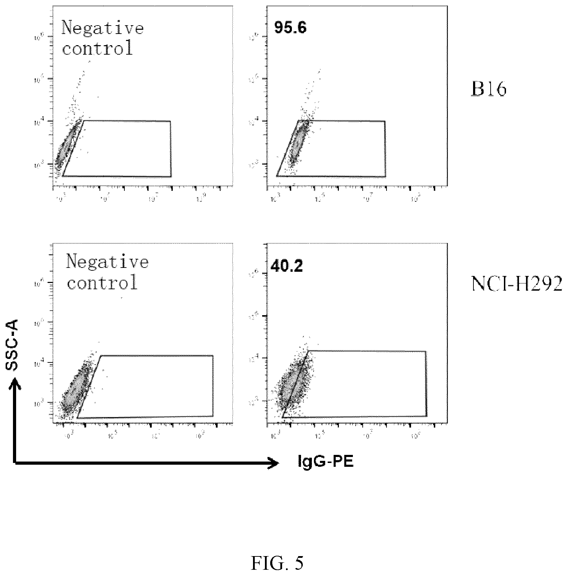

FIG. 5 shows that the monoclonal antibody Hu004-73 of the present invention can bind to human CD47 and mouse CD47 at the same time.

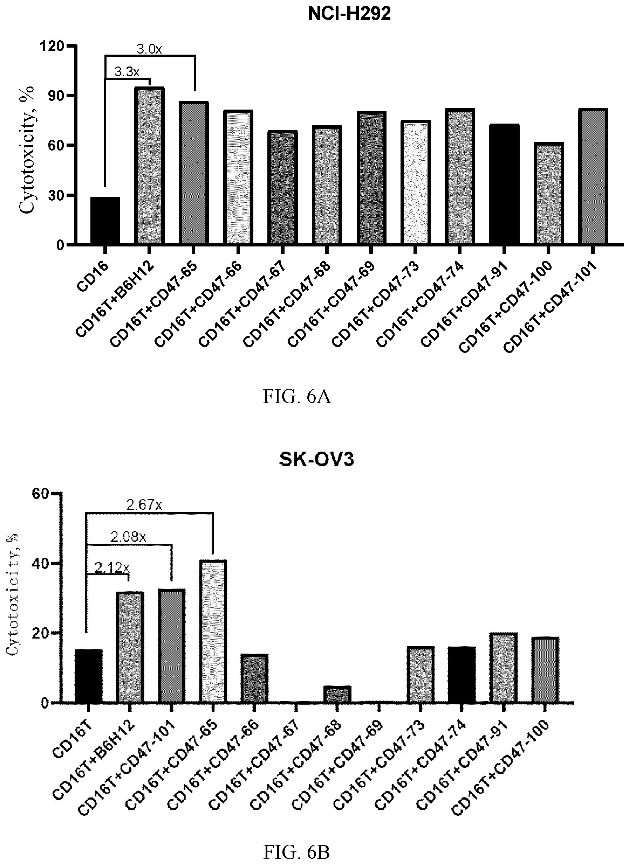

FIG. 6 shows that the monoclonal antibodies Hu004-65 and Hu004-101 of the present invention can mediate a broad-spectrum anti-tumor activity in vitro, wherein FIG. 6A shows killing activities of the candidate antibodies Hu004-65 and Hu004-101 of the present invention against an NCI-H292 tumor cell line; as can be seen from FIG. 6A, compared with other candidate antibodies, the antibodies Hu004-65 and Hu004-101 have stronger mediated killing activities in vitro, which are comparable to that of the positive control antibody B6H12; FIG. 6B shows killing activities of the candidate antibodies Hu004-65 and Hu004-101 of the present invention against an SK-OV3 tumor cell line; and as can be seen from FIG. 6B, the mediated killing activities of the candidate antibodies Hu004-65 and Hu004-101 against the SK-OV3 tumor cell line in vitro are higher than that of the positive control antibody B6H12, and both achieve twice a killing rate of the B6H12 antibody. It is indicated that the candidate antibodies Hu004-65 and Hu004-101 have mediated broad-spectrum anti-tumor activities in vitro.

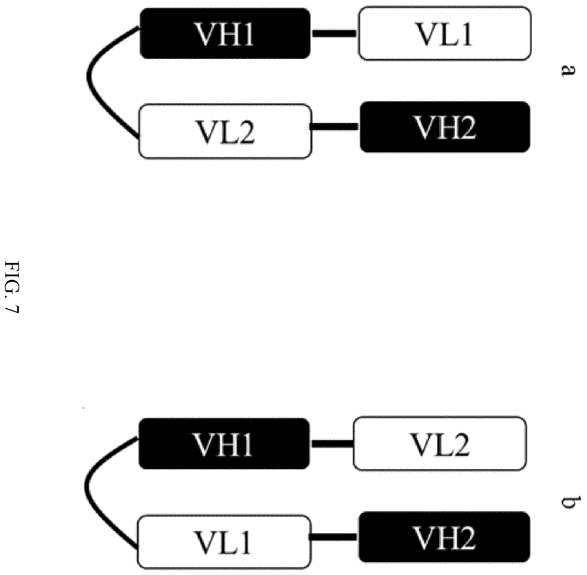

FIG. 7 shows an arrangement mode of variable regions for an antibody.

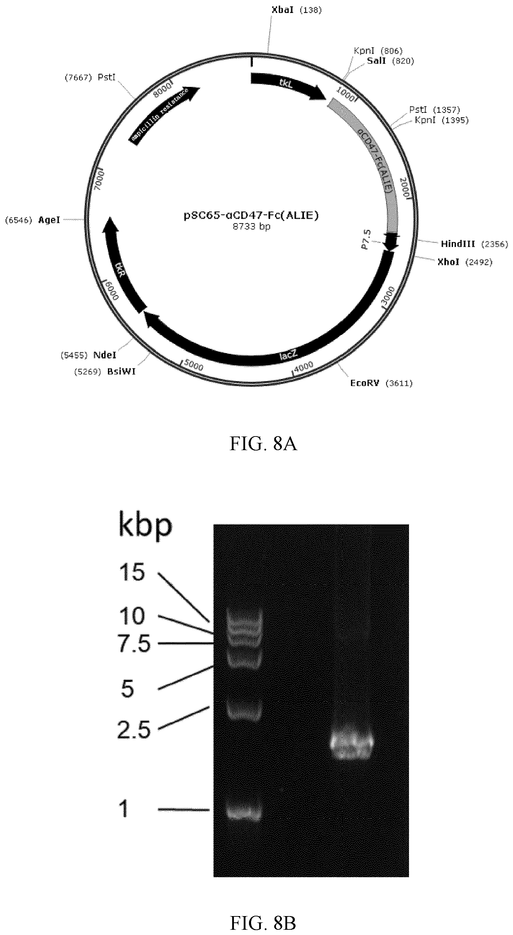

FIG. 8 shows the construction of a vaccinia virus Tian Tan strain shuttle plasmid vector αCD47-Fc(ALIE) according to a specific example of the present invention, wherein FIG. 8A shows an expression map of a vaccinia virus shuttle plasmid pSC65 integrated with an αCD47-Fc(ALIE) gene; FIG. 8B shows a PCR identification result of an insert fragment αCD47-Fc(ALIE) in the recombinant vaccinia virus rTV-αCD47-Fc(ALIE).

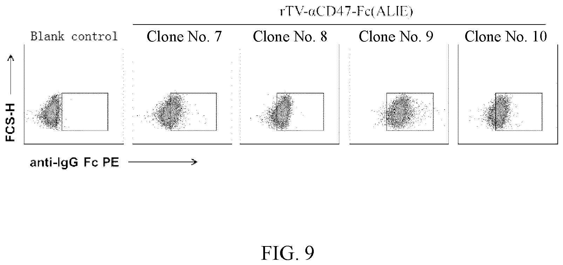

FIG. 9 shows the verification of αCD47-Fc(ALIE) protein expression in a culture supernatant after VERO cells are infected by different vaccinia virus Tian Tan strains according to an example of the present invention.

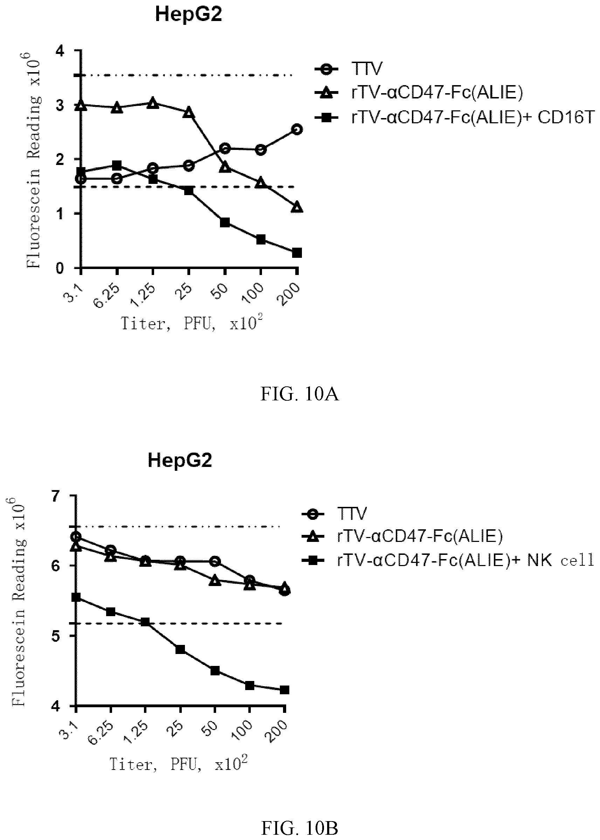

FIG. 10 shows an effect of the recombinant vaccinia virus rTV-αCD47-Fc(ALIE) against the liver cancer in vitro, wherein FIG. 10A shows in vitro killing effects of a wild-type vaccinia virus Tian Tan strain (TTV) treatment group, a hrCD16 T cell group, an αCD47-Fc(ALIE) recombinant vaccinia virus group (rTV-αCD47-Fc(ALIE)) and a rTV-αCD47-Fc(ALIE) and hrCD16 T cell combination treatment group on HepG2 cells; and FIG. 10B shows in vitro killing effects of a wild-type vaccinia virus Tian Tan strain (TTV) treatment group, an NK cell group, an αCD47-Fc(ALIE) recombinant vaccinia virus group (rTV-αCD47-Fc(ALIE)) and a rTV-αCD47-Fc(ALIE) and NK cell combination treatment group on HepG2 cells. It can be seen from FIG. 10 that a killing effect of the recombinant vaccinia virus rTV-αCD47-Fc(ALIE) on the HepG2 cells is more obvious, and is enhanced with the increase of a virus titer.

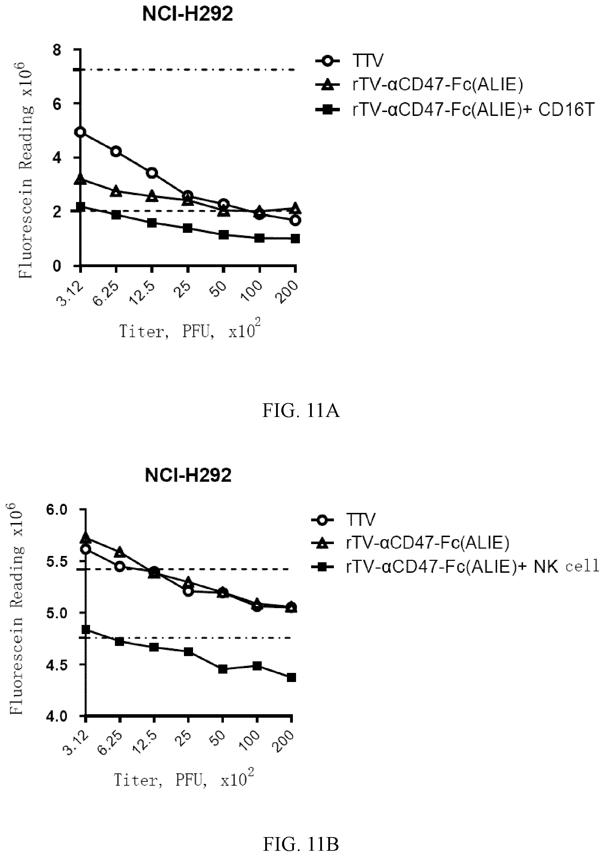

FIG. 11 shows an effect of the recombinant vaccinia virus rTV-αCD47-Fc(ALIE) against the malignant lung cancer in vitro, wherein FIG. 11A shows in vitro killing effects of a wild-type vaccinia virus Tian Tan strain (TTV) treatment group, a hrCD16 T cell group, an αCD47-Fc(ALIE) recombinant vaccinia virus group and a rTV-αCD47-Fc(ALIE) and hrCD16 T cell combination treatment group on an NCI-H292 tumor cell line; and FIG. 11B shows in vitro killing effects of a wild-type vaccinia virus Tian Tan strain (TTV) treatment group, an NK cell group, an αCD47-Fc(ALIE) recombinant vaccinia virus group and a rTV-αCD47-Fc(ALIE) and NK cell combination treatment group on the NCI-H292 tumor cell line. It can be seen from FIG. 11 that a killing effect of the recombinant vaccinia virus rTV-αCD47-Fc(ALIE) on the NCI-H292 tumor cell line is more obvious, and is enhanced with the increase of a virus titer.

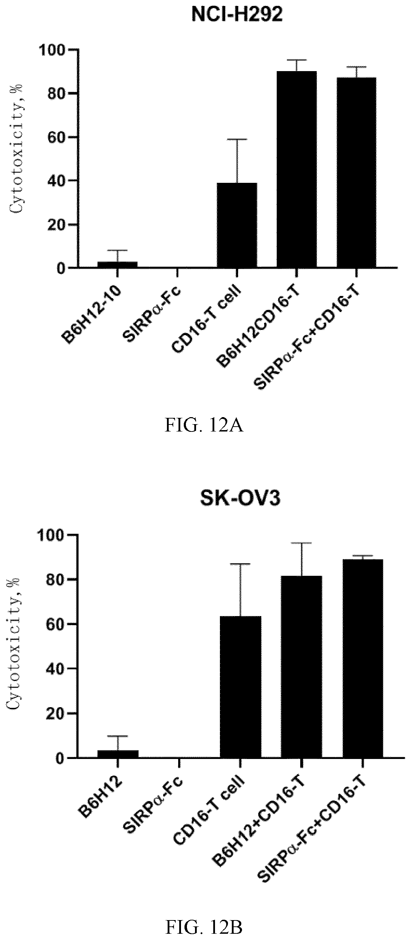

FIG. 12 shows an in vitro anti-tumor activity of SIRPα-Fc(ALIE) of the present invention, wherein FIG. 12A shows in vitro killing effects of a B6H12-Fc(ALIE) antibody treatment group alone, a SIRPα-Fc(ALIE) treatment group alone, a hrCD16 T cell group, a B6H12-Fc(ALIE) antibody and hrCD16 T cell combination treatment group, and a SIRPα-Fc(ALIE) and hrCD16 T cell combination treatment group on NCI-H292 lung cancer cells; and FIG. 12B shows in vitro killing effects of a B6H12-Fc(ALIE) antibody treatment group alone, a SIRPα-Fc(ALIE) treatment group alone, a hrCD16 T cell group, a B6H12-Fc(ALIE) antibody and hrCD16 T cell combination treatment group, and a SIRPα-Fc(ALIE) and hrCD16 T cell combination treatment group on SK-OV3 ovarian cancer cells. It can be seen from FIG. 12B that a killing effect of the SIRPα-Fc(ALIE) and hrCD16 T cell combination treatment group on SK-OV3 cells is more obvious, and is enhanced.

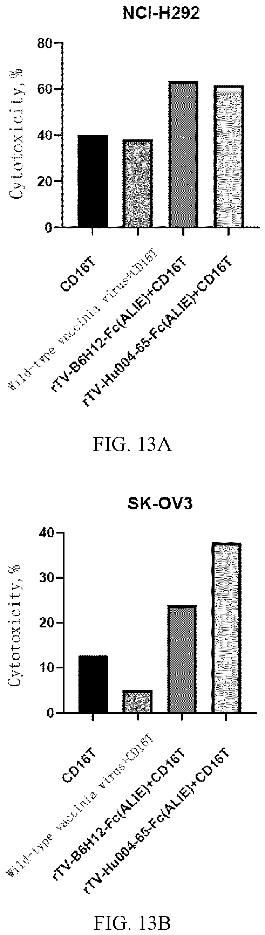

FIG. 13 shows that a rTV-Hu004-65-Fc(ALIE) poxvirus prepared from a Clone No. 65 of the present invention has an efficient anti-tumor activity in vitro. FIG. 13A shows in vitro killing effects of a hrCD16 T cell group, a wild-type vaccinia virus Tian Tan strain (TTV) and hrCD16 T cell combination treatment group, a B6H12-Fc(ALIE) recombinant vaccinia virus and hrCD16 T cell combination treatment group, and a rTV-Hu004-65-Fc(ALIE) and hrCD16 T cell combination treatment group on NCI-H292 lung cancer cells; and FIG. 13B shows in vitro killing effects of the hrCD16 T cell group, the wild-type vaccinia virus Tian Tan strain (TTV) and hrCD16 T cell combination treatment group, the B6H12-Fc(ALIE) recombinant vaccinia virus and hrCD16 T cell combination treatment group, and the rTV-Hu004-65-Fc(ALIE) and hrCD16 T cell combination treatment group on SK-OV3 ovarian cancer cells. It can be seen from FIG. 13 that the recombinant vaccinia virus rTV-Hu004-65-Fc(ALIE) poxvirus has a strong killing effect on NCI-H292 lung cancer cells and SK-OV3 ovarian cancer cells, and has a killing effect on ovarian cancer cells SK-OV3 significantly higher than that of a B6H12-Fc(ALIE) group.

### Deposit Information

The vaccinia virus Tian Tan strain is named rTV-αCD47-Fc(ALIE) and has a deposit accession number of CCTCC NO: V202080, a deposit date of January 8, 2021, and a depositary institution of China Center for Type Culture Collection (CCTCC) (address: Wuhan University, Wuhan, China).

### Mode of Carrying Out the Invention

### Example 1: Construction and screening of a native human antibody bacteriophage display library

Preparation of a human CD47 extracellular domain (hCD47-ECD) antigen: a human recombinant CD47 protein is composed of Met1-Pro139, having a total of 123 amino acid residues; and purchased from Beijing Sino Biological Inc., Lot number: 12283-H08H.

The screening of a CD47 fully human antibody was commissioned by Taki Zhan (Shanghai) Biotechnology Co., Ltd. After a total of four rounds of screening, clones obtained in the third round were selected for ELISA screening of positive clones of ELISA. Finally, a total of 25 positive clones that can bind to an hCD47-ECD protein were screened, and after sequencing analysis and ELISA binding, the sequences of 10 clones were finally selected to construct a full-length antibody for further experiments. The specific implementation method was as follows:

### 1.1 Sequencing and analysis of positive clones

After initial screening, 25 positive clones that can bind to the hCD47-ECD protein were numbered; 2 µL of a bacterial solution was pipetted into 2 mL of 2×YT medium (Sigma Aldrich, Lot number: Y1003-500ML), and cultured at 37°C, 220 rpm overnight; and plasmids were extracted for second-generation sequencing. Sequencing results were integrated and aligned with an original AB1 file through SeqMan, non-antibody gene sequences were removed from the original AB1 file, and a fasta file with an antibody gene integration version was generated. Subsequently, a DNA sequence was translated into an amino acid sequence through MEGA6, a terminator, an unconventional sequence, etc. were found through the amino acid sequence, and the fasta file containing the amino acid sequence was derived.

### 1.2 Affinity of ELISA screening and cloning supernatant binding to antigen hCD47-ECD

Firstly, the clones screened in the third round were picked up and placed in a 96-well deep-well plate containing 300 µL of 2-YT medium, and cultured at 37°C overnight, and a supernatant containing expressed Fab. The supernatant was taken, gradiently diluted, then added into an ELISA plate coated with 2 µg/mL hCD47-ECD, and then detected with horseradish peroxidase (HRP)-labeled goat anti-human Fab as a secondary antibody (ThermoFisher, Lot number: 31482) according to a dilution of 1: 6000. The higher a signal value was, and the stronger the affinity was. The results were shown in FIG. 1. The results showed that in an ELISA assay, Fabs of 10 antibodies (named a Hu004-65 antibody, a Hu004-66 antibody, a Hu004-67 antibody, a Hu004-68 antibody, a Hu004-69 antibody, a Hu004-73 antibody, a Hu004-74 antibody, a Hu004-91 antibody, a Hu004-100 antibody, and a Hu004-101 antibody respectively) showed better affinity, wherein the positive control was a B6H12 clone antibody (US 9017675B2), and the negative control was a human IgG protein.

### Example 2: Construction, expression, and purification of full-length antibody

In the present example, 10 Fab antibodies obtained in Example 1 with good activity for binding to hCD47-ECD were constructed into human IgG1 isoforms, wherein light chains were of a κ type and the antibody type was a fully human antibody.

An arrangement mode of variable regions of the antibody was shown in FIG. 7.

### 2.1 Plasmid construction

From strains containing an antibody obtained by screening, PCR amplification was used to obtain fragments of light and heavy chain variable regions of the antibody. By a homologous recombination method, the fragments of light and heavy chain variable regions of the antibody were respectively constructed to eukaryotic expression vector plasmids Cloning vector AbVec-hIgKappa (GenBank: FJ475056.1) or Cloning vector AbVec-hIgG1 (GenBank: FJ475055.1) containing light and heavy chain constant region fragments(FIG. 2) to form a complete antibody light and heavy chain full-length gene.

CDR sequences of the corresponding antibodies were shown in Table 1 below.

**Table 1 Sequences of Hu004-65, Hu004-73 and Hu004-101**

| **Antibody Nos.** | **Domains** | **Sequences** | |
|---|---|---|---|
| | | **Amino acids** | **SEQ ID NO:** |
| Hu004-65 | H-CDR | HCDR1: GYGMS | 17 |
| | | HCDR2: TITSGGTYTYYPDSVKG | 18 |
| | | HCDR3: SLAGNAMDY | 19 |
| | L-CDR | LCDR1: RASQSITKSLN | 11 |
| | | LCDR2: VASTLQS | 12 |
| | | LCDR3: QHSYS | 13 |
| | VH | EVQLVESGGGLVQPGGSLRLSCAASGFTFSG YGMSWVRQAPGKGLEWVATITSGGTYTYYP DSVKGRFTISRDNAKNSLYLQMNSLRAEDTA VYYCARSLAGNAMDYWGQGTLVTVSS | 9 |
| | VL | DIQMTQSPSSLSASVGDRVTITCRASQSITKSL NWYQQKPGKAPRLLIRVASTLQSGVPSRFSG SGSGTDFTLTISSLQPEDFATYYCQHSYSFGQ GTKLEIT | 5 |
| Hu004-101 | H-CDR | HCDR1: GYGMS | 17 |
| | | HCDR2: TITSGGTYTYYPDSVKG | 18 |
| | | HCDR3: SLAGNAMDY | 19 |
| | L-CDR | LCDR1: RASQSISNWLA | 14 |
| | | LCDR2: KASNLES | 15 |
| | | LCDR3: QQFGT | 16 |
| | VH | EVQLVESGGGLVQPGGSLRLSCAASGFTFSG YGMSWVRQAPGKGLEWVATITSGGTYTYYP DSVKGRFTISRDNAKNSLYLQMNSLRAEDTA VYYCARSLAGNAMDYWGQGTLVTVSS | 9 |
| | VL | DIQMTQSPSTLSASVGDRVTITCRASQSISNW LAWYQQKPGEAPKLLIYKASNLESGVPSRFS GSGSGTEFTLTISSLQPDDFATYYCQQFGTFG QGTKVEIK | 7 |
| Hu004-73 | H-CDR | HCDR1: GYGMS | 17 |
| | | HCDR2: TITSGGTYTYYPDSVKG | 18 |
| | | HCDR3: SLAGNAMDY | 19 |
| | L-CDR | LCDR1: RAGQPLNNYLN | 22 |
| | | LCDR2: AASTLQS | 23 |
| | | LCDR3: QHRVT | 24 |
| | VH | EVQLVESGGGLVQPGGSLRLSCAASGFTFSG | 9 |
| | | YGMSWVRQAPGKGLEWVATITSGGTYTYYP DSVKGRFTISRDNAKNSLYLQMNSLRAEDTA VYYCARSLAGNAMDYWGQGTLVTVSS | |
| | VL | DIQMTQSPSSLSASVGDRVTITCRAGQPLNNY LNWYQQKPGKPPRLLIYAASTLQSGVPSRFS GSGSGTDFTLTISGLQSEDFASYFCQHRVTFG PGTKVDVR | 25 |

### 2.2 Plasmid preparation

Constructed vectors containing the antibody light and heavy chain full-length gene were transformed into E. coli TOP10 (Weidi, Lot number: DL1010S) respectively and cultured at 37°C overnight. Endotoxin-free antibody light and heavy chain plasmids were obtained for eukaryotic expression by performing plasmid extraction with an endotoxin-free plasmid extraction kit (OMEGA, Lot number: D6950-01).

### 2.3 Expression and purification of antibodies

Candidate antibodies Hu004-65, Hu004-66, Hu004-67, Hu004-68, Hu004-69, Hu004-73, Hu004-74, Hu004-91, Hu004-100 and Hu004-101, and a control antibody B6H12 (US 9017675B2) were expressed using an Expi293 transient expression system (Thermo Fisher, Lot number: A1435101). The specific method was as follows:
On the day of transfection, the cell density was confirmed to be around 7×10⁶ to 1×10⁷ viable cells/mL and the cell viability rate was greater than 98%, at which point cells were adjusted to a final concentration of 6×10⁶ cells/mL with a fresh Expi293 expression medium which was pre-warmed at 37°C. Plasmids of interest were diluted with a Expi293^{™} Expression Medium pre-cooled at 4°C (10 µg of plasmids were added to 1 mL of the medium), and meanwhile, a FectoPro (Polyplus, Lot number: PT-116-001) transfection reagent was diluted with Expi293^{™} Expression Medium, both of which were then mixed in equal volumes, blown and beaten gently and uniformly to prepare an Expi293^{™} Expression Medium/plasmid DNA mixed solution, incubated for 15 min at room temperature, slowly added to the prepared cell suspension with gentle shaking, and finally placed in a cell culture shaker and cultured under conditions of 37 °C and 5% CO₂. After 18 to 22 h of transfection, 0.6 µL/mL FectoPRO booster was added to the culture solution. The shake flask was placed on the shaker at 37 °C and continued to culture under 5% CO₂. On day 5 after transfection, Expi293^{™} Expression Medium of the same volume was added slowly and meanwhile the cell suspension was mixed gently and uniformly. After 7 to 15 days of transfection, a cell culture supernatant in which a protein of interest was expressed was high-speed centrifuged at 4000 g for 10 min. The resulting supernatant was subjected to affinity purification with a Protein G agarose column (GE, Lot number: 28903134). The purified antibody was solubilized in PBS buffer. The protein of interest was then eluted with 100 mM sodium acetate (pH 3.0) and then neutralized with 1 M Tris-HCl. Finally, the resulting protein was replaced into the PBS buffer through an ultrafiltration concentration tube (Millipore Company, Lot number: UFC901096).

### 2.4 Concentration determination of antibodies

The relative molecular weights and purities of 10 candidate antibodies Hu004-65, Hu004-66, Hu004-67, Hu004-68, Hu004-69, Hu004-73, Hu004-74, Hu004-91, Hu004-100 and hu004-101 and a control antibody B6H12 were detected through SDS-PAGE. Concentrations of the purified antibody proteins were determined using a validated microspectrophotometer (Thermo Fisher, NanoDrop One C). A value obtained by dividing the determined A280 value by a theoretical extinction coefficient of the antibody was used as an antibody concentration value for the follow-up study. After passing the quality inspection, these antibodies were subpackaged and stored at -80°C.

### Example 3: SPR method determination of antibody affinity coefficient

Beijing Sino Biological Inc. was entrusted to perform affinity determination through surface plasmon resonance (SPR). The results were shown in FIG. 3. The results showed that the affinities of the 10 candidate antibodies were all above a 10⁻⁷ level, and all had strong affinity.

### Example 4: Candidate antibodies Hu004-65 and Hu004-101 have different recognition epitopes from B6H12 clone

A human non-small cell lung cancer A549 cell line was cultured in a DMEM medium (10% FBS, 1% antibiotic) at 37°C under 5% CO₂. Cells were collected with 0.25% trypsinization. A cell concentration was determined, the cells were centrifuged at 800 g for 3 min to discard a supernatant; and resuspended with 1 mL of 2% FBS washing buffer, and centrifuged at 800 g for 3 min to discard a supernatant; and the cells were resuspended with 100 µL of 2% FBS washing buffer. Cells were stained with the candidate antibodies Hu004-65 and Hu004-101, and a control antibody B6H12 as primary antibodies. The cells were incubated for 15 min at room temperature, resuspended with 1 mL of 2% FBS washing buffer, and centrifuged at 800 g for 3 min to discard a supernatant; added with secondary antibodies IgG-Fc-PE and IgG-Fc-APC (BioLegend, Lot numbers: 409304 and 409306), and incubated for 15 min at room temperature in dark place; resuspended with 1 mL of 2% FBS washing buffer, and centrifuged at 800 g for 3 min to discard a supernatant; and resuspended with 200 µL of medium, and tested on a machine (Beckman Coulter, CytoFLEX Flow Cytometer). The flow cytometry results were shown in FIG. 4. As shown in FIG. 4, cell populations of the positive control B6H12 and Hu004-65 dual-channel staining group, the positive control B6H12 and Hu004-101 dual-channel staining group, and the Hu004-101 and Hu004-65 dual-channel staining group were all double-positive. Therefore, it was proved that the candidate antibodies Hu004-65 and Hu004-101 had different recognition epitopes from B6H12, and the candidate antibodies Hu004-65 and Hu004-101 also had different recognition epitopes and did not compete with each other.

### Example 5: A test in which a candidate antibody Hu004-73 specifically binds to humanized CD47 and murine CD47

An NCI-H292 cell line of human lung mucoepithelioid carcinoma was cultured at 37°C under 5% CO₂ in a DMEM medium (10% FBS, 1% antibiotic). B16 melanoma cells (murine origin) were cultured in a RPMI1640 medium (10% FBS, 1% antibiotic) at 37°C under 5% CO₂. Cells were collected with 0.25% trypsinization. A cell concentration was determined, and 1E5 cells were prepared. The cells were centrifuged at 800 g for 3 min to discard a supernatant; resuspended with 1 mL of 2% FBS washing buffer, and centrifuged at 800 g for 3 min to discard a supernatant; resuspended with 100 µL of 2% FBS washing buffer, added with a primary antibody (Hu004-73) respectively, and incubated for 15 min at room temperature; resuspended with 1 mL of 2% FBS washing buffer, and centrifuged at 800 g for 3 min to discard a supernatant; added with a secondary antibody IgG-Fc-PE (BioLegend, Lot number: 409304), incubated for 15 min at room temperature in dark place; resuspended with 1 mL of 2% FBS washing buffer, and centrifuged at 800 g for 3 min to discard a supernatant; and resuspended with 200 µL of medium, and tested on a machine (Beckman Coulter, CytoFLEX Flow Cytometer). The results were shown in FIG. 5. As can be seen from FIG. 5, the specificity of Hu004-73 antibody binding to mouse melanoma cells B16 was 96.5%, and the specificity of Hu004-73 antibody binding to human lung mucoepithelioid carcinoma cells NCI-H292 was 40.2%. This experiment can demonstrate that the candidate antibody Hu004-73 was able to bind to humanized CD47 and murine CD47.

### Example 6: Test of mediated broad-spectrum anti-tumor activities of candidate antibodies Hu004-65 and Hu004-101 in vitro

In vitro mediated killing experiments of candidate antibodies were performed against SK-OV3 ovarian cancer and NCI-H292 lung cancer tumor cell lines. SK-OV3 and NCI-H292 cell lines, both of which were Luciferase-expressing cell lines, were cultured in a DMEM medium (10% FBS, 1% antibiotic) at 37°C under 5% CO₂. Tumor cells were plated in a 96-well plate at a density of 1E4/well and cultured at 37°C overnight till adherence. After 24 h, the culture was removed. It was set that 200 µL of medium was added to a blank control group, a B6H12 antibody was added to a positive control group, and candidate antibodies (Hu004-65, Hu004-66, Hu004-67, Hu004-68, Hu004-69, Hu004-73, Hu004-74, Hu004-91, Hu004-100, and Hu004-101) were added to experimental groups (Nos. 1 to 10) respectively. CD16 CAR-T cells were added to the positive control group and the experimental groups at a density of 2E4/well, respectively, and the medium was added to make the final concentration of the antibody 10 µg/mL. After 24 h, a supernatant was discarded, 50 µL of 1x cell lysate (Promega, Lot number: E1531) was added to each well, and incubated for 30 min at room temperature under shaking; and 30 µL of luciferase substrate (Promega, Lot number: E151A) was added to each well. A detection was performed on a machine (GloMax^{®} Navigator Microplate Luminometer, Promega, Steady-Glo protocol). Detection results were shown in FIG. 6. The candidate antibodies Hu004-65 and Hu004-101 had strong mediated killing activities against an NCI-H292 tumor cell line in vitro, which were comparable to that of the positive control antibody. As can be seen from FIG. 6, the mediated killing activities of the candidate antibodies Hu004-65 and Hu004-101 on the SK-OV3 tumor cell line in vitro were higher than that of the positive control antibody B6H12, and both achieved twice the killing rate of the B6H12 antibody. This experiment indicated that the candidate antibodies Hu004-65 and Hu004-101 have mediated broad-spectrum anti-tumor activities in vitro.

### Example 7: Construction and expression verification of recombinant vaccinia virus of αCD47-Fc(ALIE)

### 7.1 Construction of pSC65 vector with αCD47-Fc(ALIE) gene of interest

A DNA sequence of αCD47-Fc(ALIE) was artificially synthesized as shown in SEQ ID NO: 1, and the synthesized DNA sequence was used as a template for PCR amplification with the following primer.

The amplification primer is:
upstream: SEQ ID NO: 20
   GTACCAGGCCTAGTACTATGGAGAGGACCCTTGTCTG
downstream: SEQ ID NO: 21
   AATAAGCTCGAAGTCGACCTAGGAGAGATGCTGATG

PCR reaction procedure: pre-denaturation at 94°C for 5 min; denaturation at 98°C for 10 sec; annealing at 58°C for 30 sec; extension at 72°C for 1 min; reaction for 30 cycles; sufficient extension at 72°C for 10 min again; and termination at 25°C.

Recovery and clone construction of a PCR product: after amplification, the gene of interest was separated from 2% agarose gel, and meanwhile, a pSC65 vector was linearized through Sal I digestion (Thermo Scientific, Lot number: ER0642) and enzymatically digested gel was recycled; and PCR fragments and vector digested fragments were recycled using Sanprep Column DNA Gel Recovery Kit (Promega, Lot number: A9282). A gene recovery product was ligated to an enzymatically digested linearized vector by homologous recombination (Novozan Corporation, Lot number: C112-02). The ligated product was transformed into E. coli TOP10 and grown overnight on an ampicilin-containing plate. On the second day, a single colony was randomly selected for sequencing. A mutation site was corrected. After all sequences were verified, a pSC65 shuttle plasmid of an αCD47-Fc(ALIE) gene was successfully cloned. A plasmid construction map was shown in FIG. 8A.

### 7.2 Recombination and screening of recombinant vaccinia virus of αCD47-Fc(ALIE)

1. Cell preparation: 143TK-cells were plated in a 6-well plate, with approximately 1×10⁶ cells per well. After culture for about 24 h, when the cells are adherent and spread over the entire bottom surface, the next operation was performed.
2. Vaccinia virus incubation: cells were infected with 0.0125/3 PFU (PFU: plaque-forming unit, virus titer) of wild-type vaccinia virus Tian Tan strain, incubated in a 37°C incubator for 1 h, and then taken out; a supernatant was sucked away; and the cells were rinsed with 1 mL of PBS and then added with 1 mL of complete medium.
3. Plasmid transfection: 143TK-cells were transfected with a pSC65 shuttle plasmid with αCD47-Fc(ALIE).The cells were cultured in an incubator at 37°C for about 48 h, and the specific time depends on the conditions of cell lesions.
4. Plaque coating: a 2×DMEM maintenance medium (containing 2% PS and 4% FBS) for virus plaque coating was prepared, added with 2% pre-warmed low-melting-point agarose and then added with X-gal (a final concentration of 200 µg/mL).
5. A supernatant was sucked away from the 6-well plate and 6 mL of mixture for plaque coating was added to the 6-well plate, with 1 mL per well. Then, the 6-well plate was carefully placed in a 4°C freezer to promote coagulation. When the low-melting-point agarose was solidified, the 6-well plate was transferred to a 37°C incubator and invertedly cultured until clear blue plaques appeared.
6. Recombinant blue virus plaques were picked up and added to 500 µL of complete medium; and repeatedly frozen and thawed at -80°C for more than three times to release as much virus as possible.
7. 143TK-cells were plated in the 6-well plate, with approximately 1×10⁶ per well. After culture for about 24 h, the cells were adherent and spread over the entire bottom surface.
8. Blue plaques in an EP tube were blown and beaten repeatedly so that they were scattered completely.
9. The complete medium was replaced with a maintenance medium, then added with a virus solution containing the blue plaques and incubated in a 37°C incubator for 3 to 4 h.
10. A screening pressure was added: BrdU (50 µg/mL) was added and incubated in the 37°C incubator for about 48 h, and plaque coating was performed according to a virus plaque formation condition. This purification process needed to be performed at least 5 times.
11. Sample amplification of recombinant vaccinia virus: 143TK-cells were plated on the 6-well plate, with 1×10⁶ cells per well, and the cell density was approximately 100% of a bottom area of the well plate when used.
12. The medium in the wells was replaced with 2 mL of maintenance medium before virus seeding. The purified virus solution containing the blue plaques was blown and beaten repeatedly until the blue plaques were scattered. About 100 µL of virus solution was added to each well; and incubated in the 37°C incubator for about 48 h, and samples were collected according to a virus plaque formation condition.
13. Sample collection: 1 mL of medium supernatant was pipetted from the wells carefully. The remaining 1 mL of medium was blown off sufficiently and placed in the EP tube for subsequent PCR identification and amplification as a virus seed.

The identification results were shown in FIG. 8B, and a recombinant vaccinia virus rTV-αCD47-Fc(ALIE) was successfully inserted with an αCD47-Fc(ALIE) gene.

### 7.3 Expression verification of recombinant vaccinia virus of αCD47-Fc(ALIE)

1. Cell preparation: a 10 cm culture dish was taken and inoculated with 5×10⁶ VERO cells/dish to ensure that the cell density reaches 100% when a vaccinia virus was inoculated on the next day.
2. Virus inoculation: the complete medium was replaced with 8 mL of maintenance medium (DMEM medium + 2% FBS + 1% PS), and then added with the virus to obtain four candidate strains of the vaccinia virus, namely Clone No. 7, Clone No. 8, Clone No. 9 and Clone No. 10, which infected cells at an inoculation volume of about 0.02 MOI (MOI = virus PFU/cell count); and cultured in a 37°C, 5% CO₂ incubator for 48 h.
3. Protein purification: a cell supernatant was collected according to a virus plaque formation condition, and an antibody was enriched with a Protein G agarose column (GE, Lot number: 28-9031-34), and dissolved in PBS buffer.
4. CD47 antibody affinity assay: the purified protein was incubated with A549 cells (1×10⁶) at a concentration of 40 µg/mL for 15 min at room temperature, and a protein sample without αCD47-Fc(ALIE) was used as a blank control. 1 mL of PBS (FPBS) with 2% FBS was used for washing twice; each sample was incubated together with 1 µL of PE-labeled anti-human IgG Fc antibody (BioLegend, Lot number: 409304) for 15 min at room temperature, and washed twice with 1 mL of FPBS; and cells were resuspended with 200 µL of FBS for detection on a machine.

The results were shown in FIG. 9. Compared with the blank control, in the four vaccinia virus groups, a binding rate of αCD47-Fc(ALIE) expressed and secreted by Clone No. 9 to A549 cells was about 95%. The results indicated that a CD47 antibody gene carried by the recombinant vaccinia virus not only successfully expressed the CD47 antibody, but also had better binding ability to the CD47 protein on the A549 cells. The Clone No. 9 was deposited and named rTV-αCD47-Fc(ALIE) and had a deposit accession number of CCTCC NO: V202080, a deposit date of January 8, 2021, and a depositary institution of China Center for Type Culture Collection (CCTCC) (address: Wuhan University, Wuhan, China).

### Example 8: Amplification and titer determination of recombinant vaccinia virus of αCD47-Fc(ALIE)

### 8.1 Amplification and purification of recombinant vaccinia virus of αCD47-Fc(ALIE)

1. VERO cell plating: each 10 cm dish was inoculated with 5×10⁶ cells to ensure that the cell density reached 100% when a vaccinia virus was inoculated on the next day.
2. Before virus inoculation, the complete medium was replaced with 8 mL of maintenance medium (DMEM medium + 2% FBS + 1% PS), the virus (0.02 MOI) was inoculated and continued to be cultured in a 37°C, 5% CO₂ incubator for about 48 h, and a sample was collected according to a virus plaque formation condition.
3. Vaccinia virus collection: the medium in the dish was discarded, 2 mL of maintenance medium was taken to blow off the remaining cells, which were then collected in a 15 mL centrifuge tube.
4. After frozen storage for 24 h, a virus solution was frozen and thawed repeatedly twice, subjected to density gradient centrifugation with a 36% sucrose solution, and centrifuged at 4°C for 90 min at 16000 g; a supernatant was carefully discarded; and a virus precipitate in the centrifuge tube was dissolved with PBS buffer, and subpackaged and stored at -80°C for the determination of the virus titer.

### 8.2 Titer determination of recombinant vaccinia virus of αCD47-Fc(ALIE).

1. Preparation of 143TK-cells: 143TK-cells were plated on a 24-well plate, with 2×10⁵ cells per well, and the cell density reached 100% of a bottom area of the 24-well plate in use.
2. Virus dilution: the vaccinia virus solution was diluted with a maintenance medium, starting at 1: 100 from 10-fold dilution to a final volume of 1100 µL.
3. The complete medium in the 24-well plate was discarded, and added with 500 µL of diluted virus solution, and duplicate wells were set for each sample. The incubation was performed in the 37°C, 5% CO₂ incubator for about 48 h, and the plaque coating time was determined according to a virus plaque formation condition.
4. Preparation of plaque coating gel: 8 mL of plaque coating medium containing 2×DMEM medium+4% FBS+2% PS and 8 mL of low-melting-point agarose also in a 37°C water bath after melting in a boiling water bath were mixed to obtain a mixture, and X-gal was added to the mixture (200 µg/mL) for later use.
5. The supernatant was sucked away from the 24-well plate. The plaque coating mixture in (4) was immediately added to the 24-well plate at 500 µL per well. Then, the 24-well plate was carefully placed in a 4°C freezer to promote coagulation. The low-melting-point agarose was solidified, then transferred to a 37°C incubator and invertedly cultured until clear blue plaques appeared.
6. Virus plaque counting: firstly, whether the quantity of virus plaques was decreased by a 10-fold ratio was observed, the quantity of single-digit blue plaques in the two duplicate wells for virus seeding was counted subsequently, and a sum of the quantities of the blue plaques in the two wells was obtained and multiplied by a reciprocal value of the corresponding dilution of the wells to obtain the titer of 1 mL of the virus.

### Example 9: Effect of recombinant vaccinia virus of αCD47-Fc(ALIE) in vitro killing of liver cancer cells

1. Experimental grouping was as follows:
   A: A wild-type vaccinia virus Tian Tan strain (TTV) treatment group, a hrCD16 T cell group, an αCD47-Fc(ALIE) recombinant vaccinia virus group (rTV-αCD47-Fc(ALIE)), and a rTV-αCD47-Fc(ALIE) and hrCD16 T cell combination treatment group.
   B: A wild-type vaccinia virus Tian Tan strain (TTV) treatment group, a NK cell group, an αCD47-Fc(ALIE) recombinant vaccinia virus group (rTV-αCD47-Fc(ALIE)), and a rTV-αCD47-Fc(ALIE) and NK cell combination treatment group.
   hrCD16 T cells were primary T cells modified by a variant hrCD16 chimeric receptor, and can efficiently bind to Fc (mutationally modified) of the CD47 antibody, thereby mediating targeted killing of tumor cells by T cells. NK cells were amplified and sorted from human PBMCs.
2. Preparation of HepG2 cells: HepG2 was a stable transfection cell line (Shanghai Sinobay Biotechnology Co., Ltd.) overexpressing luciferase; HepG2 cells were plated on a 96-well plate, with 1×10⁴ cells per well; and the cell density reached 100% of a bottom area of the 96-well plate in use.
3. Virus inoculation: in the recombinant vaccinia virus group and the combination treatment group, HepG2 cells were infected with the recombinant vaccinia virus (rTV-αCD47-Fc(ALIE)) for 12 h. The virus was infected in 2-fold increments of 7 doses from 3.125×10² PFU to 2×10⁴ PFU. At the same time, HepG2 cells infected with the wild-type vaccinia virus (TTV) were used as a control.
4. After 12 h of infection, 1×10⁴ hrCD16 T cells were added to the hrCD16 T cell group and the combination treatment group and continued to be cultured for 12 h; or, after 12 h of infection, 1×10⁴ NK cells were added to the NK cell group and the combination treatment group and continued to be cultured for 12 h.
5. A cell supernatant was discarded, and luciferase detection was performed using Luciferase Assay System (Promega, Lot number: E1501). The specific method was as follows: a supernatant was discarded; and 50 µL of 1×cell lysis buffer was added to each well, incubated in a shaker at room temperature for 30 min, and added with 30 µL of substrate for detection on a machine (in dark place).

The results were shown in FIG. 10. Fluorescence values measured in the rTV-αCD47-Fc(ALIE) group and the combination treatment group were decreased with the increase of the virus titer, indicating that the killing effect on the HepG2 cells was enhanced with the increase of the virus titer. Compared with the addition of the virus alone or of hrCD16T cells or NK cells, the rTV-αCD47-Fc(ALIE) and hrCD16T or NK cell combination group can significantly improve the killing of HepG2 cells in vitro.

### Example 10: Effect of recombinant vaccinia virus of αCD47-Fc(ALIE) against lung cancer in vitro

1. Experimental grouping was as follows:
   A: A wild-type vaccinia virus Tian Tan strain (TTV) treatment group, a hrCD16 T cell group, an αCD47-Fc(ALIE) recombinant vaccinia virus group, and a rTV-αCD47-Fc(ALIE) and hrCD16 T cell combination treatment group.
   B: A wild-type vaccinia virus Tian Tan strain (TTV) treatment group, a NK cell group, an αCD47-Fc(ALIE) recombinant vaccinia virus group, and a rTV-αCD47-Fc(ALIE) and NK cell combination treatment group.
2. Preparation of NCI-H292 tumor cell line: the NCI-H292 tumor cell line was a stable transfection cell line (Shanghai Sinobay Biotechnology Co., Ltd.) overexpressing luciferase; NCI-H292 cells were plated on a 96-well plate, with 1×10⁴ cells per well; and the cell density reached 100% of a bottom area of the 96-well plate in use.
3. Virus inoculation: in the recombinant vaccinia virus group and the combination treatment group, NCI-H292 cells were infected with the recombinant vaccinia virus (rTV-αCD47-Fc(ALIE)) for 12 h. The virus was infected in 2-fold increments of 7 doses from 3.125×10² PFU to 2×10⁴ PFU. At the same time, the wild-type vaccinia virus (TTV)-infected NCI-H292 cells were used as a control.
4. After 12 h of infection, 1×10⁴ hrCD16 T cells were added to the hrCD16 T cell group and the combination treatment group and continued to be cultured for 12 h; or after 12 h of infection, 1×10⁴ NK cells were added to the NK cell group and the combination treatment group and continued to be cultured for 12 h.
5. A cell supernatant was discarded, and luciferase detection was performed using Luciferase Assay System (Promega, Lot number: E1501). The specific method was as follows: a supernatant was discarded; and 50 µL of 1×cell lysis buffer was added to each well, incubated in a shaker at room temperature for 30 min, and added with 30 µL of substrate for detection on a machine (in dark place).
6. The results were shown in FIG. 11. With the increase of the virus titer, the fluorescence values of the wild-type vaccinia virus Tian Tan strain (TTV) group, the rTV-αCD47-Fc(ALIE) group, and the rTV-αCD47-Fc(ALIE) and hrCD16 T cell or NK cell combination treatment group were decreased, indicating that the killing effect on the NCI-H292 tumor cell line was enhanced. Compared with the addition of a virus alone or of hrCD16T cells or NK cells, the rTV-αCD47-Fc(ALIE) and hrCD16T or NK cell combination group can significantly enhance the killing for the NCI-H292 tumor cell line.
7. In summary, as an oncolytic virus, the recombinant vaccinia virus rTV-αCD47-Fc(ALIE) can significantly inhibit the growth of various solid tumor cells such as human liver cancer and lung cancer, and had very high application value for the treatment of tumors. The oncolytic virus was simple to prepare and convenient for mass preparation and popularization.

### Example 11: Mediated broad-spectrum anti-tumor activity test of SIRPα-Fc(ALIE) protein in vitro

The SIRPα-Fc(ALIE) antibody (having a sequence as shown in SEQ ID NO: 3 or 4) of the present invention was used to perform a mediated killing experiment in vitro for SK-OV3 ovarian cancer and NCI-H292 lung cancer tumor cell lines. SK-OV3 and NCI-H292 cell lines, both of which were Luciferase-expressing cell lines, were cultured in a DMEM medium (10% FBS, 1% antibiotic) at 37°C under 5% CO₂. Tumor cells were plated in a 96-well plate at a density of 1E4/well and cultured at 37°C overnight till adherence. After 24 h, the medium was removed, and it was set that 200 µL of medium was added to a blank control group, a B6H12 antibody was added to a positive control group, and SIRPα-Fc(WT) and SIRPα-Fc(ALIE) were added to the experimental groups respectively. CD16 CAR-T cells were added to the positive control group and the experimental groups at a density of 2E4/well, respectively, and the medium was added to make the final concentration of the antibody 10 µg/mL. After 24 h, a supernatant was discarded, 50 µL of 1x cell lysate (Promega, Lot number: E1531) was added to each well, and incubated for 30 min at room temperature; and 30 µL of luciferase substrate (Promega, Lot number: E151A) was added to each well. A detection was performed on a machine (GloMax^{®} Navigator Microplate Luminometer, Promega, Steady-Glo protocol). Detection results were shown in FIG. 12. The SIRPα-Fc(ALIE) antibody had strong mediated killing activities against the NCI-H292 tumor cell line in vitro, which was comparable to that of the positive control antibody. As can be seen from FIG. 12, the mediated killing activity of the SIRPα-Fc(ALIE) antibody against the SK-OV3 tumor cell line in vitro was higher than that of the positive control antibody B6H12, and the killing rate of the SIRPα-Fc(ALIE) antibody was 1.2-fold higher than that of the B6H12 antibody. This experiment indicated that the SIRPα-Fc(ALIE) antibody also had a mediated broad-spectrum anti-tumor activity in vitro.

### Example 12: Mediated broad-spectrum anti-tumor activity test of rTV-Hu004-65-Fc(ALIE) poxvirus in vitro

The Hu004-65-Fc(ALIE) antibodies (having sequences as shown in SEQ ID NOs: 5 and 9) of the present invention were loaded in a poxvirus vector and used to perform a mediated killing experiment in vitro against SK-OV3 ovarian cancer and NCI-H292 lung cancer tumor cell lines. SK-OV3 and NCI-H292 cell lines, both of which were Luciferase-expressing cell lines, were cultured in a DMEM medium (10% FBS, 1% antibiotic) at 37°C under 5% CO₂. Tumor cells were plated in a 96-well plate at a density of 1E4/well and cultured at 37°C overnight till adherence. After 24 h, the medium was removed, and it was set that 200 µL of medium was added to a blank control group, the supernatant infected with a wild-type virus was added to a virus control group, a culture supernatant of the cells infected with rTV-B6H12-Fc(ALIE) was added to a positive control group, and the supernatant infected with rTV-Hu004-65-Fc(ALIE) poxvirus was added to the experimental groups. CD16 CAR-T cells were added to the virus control group, the positive control group and the experimental groups at a density of 2E4/well, respectively. After 24 h, a supernatant was discarded, 50 µL of 1x cell lysate (Promega, Lot number: E1531) was added to each well, and incubated for 30 min at room temperature under shaking; and 30 µL of luciferase substrate (Promega, Lot number: E151A) was added to each well. A detection was performed on a machine (GloMax^{®} Navigator Microplate Luminometer, Promega, Steady-Glo protocol). Detection results were shown in FIG. 13. The rTV-Hu004-65-Fc(ALIE) had strong mediated killing activity against the NCI-H292 tumor cell line in vitro, which was comparable to that of a positive control antibody. As can be seen from FIG. 13, the mediated killing activity of rTV-Hu004-65-Fc(ALIE) on the SK-OV3 tumor cell line in vitro was higher than that of a positive control rTV-B6H12-Fc(ALIE), and the killing rate of rTV-Hu004-65-Fc(ALIE) was 1.6-fold higher than that of rTV-B6H12-Fc(ALIE). This experiment indicated that the poxvirus rTV-Hu004-65-Fc(ALIE), prepared from the candidate antibody Hu004-65-Fc(ALIE), also had the ability to mediate the broad-spectrum antitumor activity in vitro.

The above examples are exemplary, and should not be construed as limiting the present invention. A person of ordinary skill in the art can make changes, modifications, substitutions and variations to the above examples within the scope of the present invention.

## Claims

1. An antibody capable of specifically binding to CD47 or an antigen binding fragment thereof, the antibody or the antigen binding fragment comprising:
(a) a heavy chain variable region comprising the following three complementary determining regions:
(i) VH CDR1, which consists of the following sequence: SEQ ID NO: 17, or a sequence having one or more amino acid substitutions, deletions, or additions compared thereto;
(ii) VH CDR2, which consists of the following sequence: SEQ ID NO: 18, or a sequence having one or more amino acid substitutions, deletions, or additions compared thereto; and
(iii) VH CDR3, which consists of the following sequence: SEQ ID NO: 19, or a sequence having one or more amino acid substitutions, deletions, or additions compared thereto;
and/or
(b) a light chain variable region comprising the following three complementary determining regions:
(iv) VL CDR1, which consists of any one of the following sequences: SEQ ID NO: 11, SEQ ID NO: 14, or SEQ ID NO: 22, or a sequence having one or more amino acid substitutions, deletions, or additions compared thereto;
(v) VL CDR2, which consists of any one of the following sequences: SEQ ID NO: 12, SEQ ID NO: 15, or SEQ ID NO: 23, or a sequence having one or more amino acid substitutions, deletions, or additions compared thereto; and
(vi) VL CDR3, which consists of any one of the following sequences: SEQ ID NO: 13, SEQ ID NO: 16, or SEQ ID NO: 24, or a sequence having one or more amino acid substitutions, deletions, or additions compared thereto;
preferably, the substitution in any one of (i) to (vi) is a conservative substitution;
preferably, the VH in the antibody or the antigen binding fragment thereof comprises: VH CDR1 as shown in SEQ ID NO: 17, VH CDR2 as shown in SEQ ID NO: 18, and VH CDR3 as shown in SEQ ID NO: 19; and
the VL in the antibody or the antigen binding fragment thereof comprises: VL CDR1 as shown in SEQ ID NO: 11, VL CDR2 as shown in SEQ ID NO: 12, and VL CDR3 as shown in SEQ ID NO: 13; or
the VL in the antibody or the antigen binding fragment thereof comprises: VL CDR1 as shown in SEQ ID NO: 14, VL CDR2 as shown in SEQ ID NO: 15, and VL CDR3 as shown in SEQ ID NO: 16; or
the VL in the antibody or the antigen binding fragment thereof comprises: VL CDR1 as shown in SEQ ID NO: 22, VL CDR2 as shown in SEQ ID NO: 23, and VL CDR3 as shown in SEQ ID NO: 24.

2. An antibody capable of specifically binding to CD47 or an antigen binding fragment thereof, the antibody or the antigen binding fragment comprising a heavy chain variable region and a light chain variable region, wherein:
the heavy chain variable region comprises three CDRs contained in the heavy chain variable region shown in SEQ ID NO: 9; and the light chain variable region comprises three CDRs contained in the light chain variable region shown in SEQ ID NO: 5, 7, or 25; and
preferably, the three CDRs contained in the heavy chain variable region, and/or the three CDRs contained in the light chain variable region, are defined by a Kabat, Chothia, or IMGT numbering system.

3. The antibody or the antigen binding fragment thereof according to claim 1 or 2, wherein the antibody or the antigen binding fragment thereof comprises:
(a) a heavy chain variable region comprising an amino acid sequence selected from the group consisting of:
(i) a sequence shown in SEQ ID NO: 9;
(ii) a sequence having one or more amino acid substitutions, deletions, or additions compared to the sequence shown in SEQ ID NO: 9; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence shown in SEQ ID NO: 9;
and/or,
(b) a light chain variable region comprising an amino acid sequence selected from the group consisting of:
(iv) a sequence shown in any one of SEQ ID NO: 5, SEQ ID NO: 7, or SEQ ID NO: 25;
(v) a sequence having one or more amino acid substitutions, deletions, or additions compared to the sequence shown in any one of SEQ ID NO: 5, SEQ ID NO: 7, or SEQ ID NO: 25; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence shown in any one of SEQ ID NO: 5, SEQ ID NO: 7, or SEQ ID NO: 25;
preferably, the substitution in (ii) or (v) is a conservative substitution;
preferably, the antibody or the antigen binding fragment thereof comprises: a VH having a sequence as shown in SEQ ID NO: 9 and a VL having a sequence as shown in any one of SEQ ID NO: 5, 7, or 25.

4. The antibody or the antigen binding fragment thereof according to any one of claims 1 to 3, wherein the antibody or the antigen binding fragment thereof further comprises:
(a) a heavy chain constant region of a human immunoglobulin or a variant thereof, the variant having one or more amino acid substitutions, deletions, or additions compared to a sequence from which the variant is derived; and
(b) a light chain constant region of a human immunoglobulin or a variant thereof, the variant having conservative substitutions of up to 20 amino acids compared to a sequence from which the variant is derived;
preferably, the heavy chain constant region is an IgG heavy chain constant region, more preferably an IgG1, IgG2, IgG3, or IgG4 heavy chain constant region, further preferably, a human IgG1 or human IgG4 heavy chain constant region; and
preferably, the light chain constant region is a κ light chain constant region.

5. The antibody or the antigen binding fragment thereof according to any one of claims 1 to 4, wherein the antigen binding fragment is selected from the group consisting of Fab, Fab', (Fab')₂, Fv, disulfide-bonded Fv, scFv, a double antibody, and a single-domain antibody; and/or, the antibody is a murine antibody, a chimeric antibody, a humanized antibody, a bispecific antibody, or a multi-specific antibody; more preferably, the antibody is a fully human antibody.

6. An isolated nucleic acid molecule, which encodes the antibody or the antigen binding fragment thereof according to any one of claims 1 to 5, or a heavy chain variable region and/or a light chain variable region thereof; and
preferably, the nucleic acid molecule comprises a nucleic acid sequence shown in any one of SEQ ID NO: 6, 8, 10, or 26.

7. A vector, comprising the isolated nucleic acid molecule according to claim 6;
preferably, the vector is a cloning vector or an expression vector;
more preferably, the vector is a virus; and
further preferably, the vector is a cloning vector AbVec-hIgKappa or a cloning vector AbVec-hIgG1. .

8. A host cell, comprising the isolated nucleic acid molecule according to claim 6 or the vector according to claim 7, wherein
preferably, the host cell is prokaryotic or eukaryotic; more preferably, the host cell is selected from an E. coli cell, a yeast cell, a mammalian cell, or other cells suitable for the preparation of an antibody or antigen binding fragment or a multi-specific antibody; further preferably, the host cell is a mammalian cell; further preferably, the host cell is a cell derived from human beings, mouse, sheep, horse, dog, or cat; and most preferably, the host cell is a 293 cell or a CHO cell.

9. A method for preparing the antibody or the antigen binding fragment thereof according to any one of claims 1 to 5, comprising: culturing the host cell according to claim 8 under a condition that allows the expression of the antibody or the antigen binding fragment thereof according to any one of claims 1 to 5; and recycling the antibody or the antigen binding fragment thereof from the cultured host cell culture.

10. A recombinant oncolytic virus, which is operably inserted with or comprises a gene coding sequence of an anti-CD47 antibody or a CD47 ligand; and
preferably, the gene coding sequence is located in a thymidine kinase region of the recombinant oncolytic virus.

11. The recombinant oncolytic virus according to claim 10, wherein the gene coding sequence of the anti-CD47 antibody or the CD47 ligand is expressed alone or in fusion with other genes or fragments;
preferably, other genes or fragments for fusion expression are one or more selected from a Fc fragment, a chemokine CXCL9, CXCL10, CXCL11, CXCL12, CCL20 or CX3CL1, or a cholera toxin CTA or CTB;
preferably, the recombinant oncolytic virus further comprises a gene coding sequence of other immunoregulatory factors; more preferably, the other immunoregulatory factors are one or more selected from IL-1, IL-2, IL-3, IL-7, IL-11, IL-12, IL-15, IL-17, IL-18, IL-21, IL-33, IL-35, IL-37, GM-CSF, IFN-α, IFN-β, IFN-γ, an anti-PD-1/PD-L1 antibody, an anti-CTLA-4 antibody, an anti-Lag-3 antibody, an anti-TIGIT antibody, or an anti-Tim-3 antibody;
preferably, the recombinant oncolytic virus further comprises a gene coding sequence of a protein related to apoptosis and pyroptosis; more preferably, the protein related to apoptosis and pyroptosis is one or more selected from an apoptosis-related factor 1, an interleukin-1β converting enzyme, a Bcl-2 protein, Fas/APO-1, p53, myc, an ataxia telangiectasia mutant gene, gasdermin D, or gasdermin E; or
preferably, the recombinant oncolytic virus further comprises small RNAs of an immunoregulatory gene, an apoptosis gene, and a pyroptosis gene.

12. The recombinant oncolytic virus according to claim 10 or 11, wherein the anti-CD47 antibody comprises the antibody or the antigen binding fragment thereof according to any one of claims 1 to 5; preferably, the anti-CD47 antibody further comprises an Fc mutant with an A330L/I332E mutation;
preferably, the anti-CD47 antibody has a sequence identity of at least 90%, 91%, 92%, 93%, 94%, 95%, 96%, 97%, 98%, or 99% with the sequence shown in SEQ ID NO: 2; more preferably, the amino acid sequence of the anti-CD47 antibody is shown in SEQ ID NO: 2; and
preferably, the CD47 ligand is an extracellular domain of SIRPα, or a functional fragment or variant thereof; more preferably, the anti-CD47 ligand has a sequence identity of at least 90%, 91%, 92%, 93%, 94%, 95%, 96%, 97%, 98%, or 99% with the sequence shown in SEQ ID NO: 4; and further preferably, the amino acid sequence of the anti-CD47 ligand is shown in SEQ ID NO: 4.

13. The recombinant oncolytic virus according to claim 10 or 11, wherein the oncolytic virus comprises a gene coding sequence having a sequence identity of at least 90%, 91%, 92%, 93%, 94%, 95%, 96%, 97%, 98%, or 99% with the sequence shown in SEQ ID NO: 1 or 3; and
preferably, the oncolytic virus comprises the gene coding sequence as shown in SEQ ID NO: 1 or 3.

14. The recombinant oncolytic virus according to any one of claims 10 to 13, wherein a virus skeleton of the oncolytic virus is derived from a modified or engineered vaccinia virus Tian Tan strain, a vaccinia virus New York strain, a vaccinia virus Copenhagen strain, a vaccinia virus canary strain, a vaccinia virus Ankara strain, an adenovirus, an adeno-associated virus, a herpes simplex virus, a varicella-zoster virus, a respiratory syncytial virus, a Semliki forest virus, an EB virus, a cytomegalovirus, a human Herpesvirus 6, a smallpox virus, a vaccinia virus, a molluscum contagiosum virus, an Orfvirus, a reovirus, a rotavirus, an enterovirus, a Seneca virus, a poliovirus, a coxsackievirus, a rhinovirus, a hepatitis A virus, a foot and mouth disease virus, a togavirus, an alphavirus, a Semliki forest virus, an eastern equine encephalitis virus, a Sindbis virus, a rubella virus, a coronavirus, a flavivirus, a hepatitis C virus, a Japanese Encephalitis virus, a St. Louis encephalitis virus, a Murray Valley fever virus, a yellow fever virus, a West Nile virus, a Zika virus, a dengue virus, an Ebola virus, a Marburg virus, an arenavirus, a Lassa fever virus, a lymphocytes Choriomeningitis virus, a Pichinde virus, a Junin virus, Machupo virus, a Hantaan virus, a Rift Valley fever virus, a Paramyxovirus, a human parainfluenza virus, a Mumps virus, a simian virus 5, a measles virus, a vesicular stomatitis virus, a rabies virus, an orthomyxovirus, an influenza A virus, an influenza B virus, an influenza C virus, a hepatitis D virus, a simian immunodeficiency virus, a human immunodeficiency virus 1, a human immunodeficiency virus 2, a Rous sarcoma virus, a human T-cell leukemia virus 1, a simian foamy virus, a hepatitis B virus, hepatitis E virus, a human papillomavirus, or a polyomavirus; and
preferably, the oncolytic virus skeleton is an intracellular mature virus, an intracellular packaging virus, a cell-associated packaging virus, or an extracellular packaging virus.

15. A recombinant vaccinia virus Tian Tan strain, named rTV-αCD47-Fc(ALIE), and having a deposit accession number of CCTCC NO: V202080.

16. A method for preparing the recombinant oncolytic virus according to any one of claims 10 to 15, comprising the steps of:
1) synthesizing a sequence including a gene coding sequence of an anti-CD47 antibody or a CD47 ligand;
2) cloning the coding sequence obtained in step 1) into a shuttle plasmid of the oncolytic virus to construct a recombinant plasmid vector;
3) transfecting the recombinant plasmid vector obtained in step 2) into the oncolytic virus, and obtaining the recombinant oncolytic virus through screening; and
optionally, culturing the obtained recombinant oncolytic virus.

17. The method according to claim 16, comprising the steps of:
1) synthesizing a human gene αCD47-Fc(ALIE), having a sequence as shown in SEQ ID NO: 1; or
comprising therein a nucleic acid sequence shown in any one of SEQ ID NO: 6, 8, 10, or 26; or
synthesizing a human gene SIRPα-Fc(ALIE), having a sequence as shown in SEQ ID NO: 3;
2) subcloning the synthesized αCD47-Fc(ALIE) gene or SIRPα-Fc(ALIE) gene into a TK region of a vaccinia virus shuttle plasmid pSC65 to construct a recombinant plasmid pSC65-αCD47-Fc(ALIE) or pSC65-SIRPα-Fc(ALIE); and
3) transfecting the pSC65-αCD47-Fc(ALIE) plasmid or the pSC65-SIRPα-Fc(ALIE) plasmid into TK143-cells that have been infected with a wild-type vaccinia virus by means of gene homologous recombination, and homologously recombining the both to produce a recombinant vaccinia virus rTV-αCD47-Fc(ALIE) or rTV-SIRPα-Fc(ALIE); and obtaining through screening a recombinant oncolytic vaccinia virus, of which the TK region includes a coding sequence of pSC65-αCD47-Fc(ALIE) shown in SEQ ID NO: 1, or a recombinant oncolytic vaccinia virus, of which the TK region includes a coding sequence of pSC65-SIRPα-Fc(ALIE) shown in SEQ ID NO: 3; and
preferably, the αCD47-Fc(ALIE) gene or the SIRPα-Fc(ALIE) gene is controlled by an early/late promoter p7.5 of the vaccinia virus.

18. Use of the recombinant oncolytic virus according to any one of claims 10 to 17 in the preparation of an anti-tumor medicament, wherein
preferably, the tumor is one or more selected from B-cell lymphoma; T-cell lymphoma; melanoma; prostatic cancer; renal cell carcinoma; sarcoma; glioma, preferably high-grade glioma; blastoma, preferably neuroblastoma; osteosarcoma; plasmacytoma; histiocytoma; pancreatic cancer; breast cancer; lung cancers such as small cell lung cancer and non-small cell lung cancer; gastric cancer; liver cancer; colon cancer; rectal cancer; esophageal cancer; colorectal cancer; hematopoietic system cancer; testicular cancer; cervical cancer; ovarian cancer; bladder cancer; squamous cell carcinoma; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or blood neoplastic disorders.
